# EUROPÄISCHE PATENTSCHRIFT

(11) **EP 0 790 988 B1**
(45) Veröffentlichungstag und Bekanntmachung des Hinweises auf die Patenterteilung: **15.01.2003**
(21) Anmeldenummer: 95936571.9
(22) Anmeldetag: 30.10.1995
(51) Int. Cl.: C07D 265/32, C07D 498/04, C07C 229/12, C07C 271/22, A61K 31/535

(54) **VERWENDUNG VON DIOXOMORPHOLINEN ZUR BEKÄMPFUNG VON ENDOPARASITEN, NEUE DIOXOMORPHOLINE UND VERFAHREN ZU IHRER HERSTELLUNG**
USE OF DIOXOMORPHOLINES TO COMBAT ENDOPARASITES, NOVEL DIOXOMORPHOLINES AND PROCESS FOR THEIR PRODUCTION
UTILISATION DE DIOXOMORPHOLINES POUR LUTTER CONTRE LES ENDOPARASITES, NOUVELLES DIOXOMORPHOLINES ET LEUR PROCEDE DE PRODUCTION

(30) Priorität: 10.11.1994 DE 4440193
(43) Veröffentlichungstag der Anmeldung: 27.08.1997
(73) Patentinhaber: BAYER AG, 51368 Leverkusen (DE)
(72) Erfinder: PLANT, Andrew, D-51373 Leverkusen (DE); SCHERKENBECK, Jürgen, D-42929 Wermelskirchen (DE); JESCHKE, Peter, D-51373 Leverkusen (DE); HARDER, Achim, D-51109 Köln (DE); MENCKE, Norbert, D-51381 Leverkusen (DE)
(86) Internationale Anmeldenummer: EP9504255
(87) Internationale Veröffentlichungsnummer: WO96015121

(56) Entgegenhaltungen:
- EP-A- 0 626 375
- EP-A- 0 626 376
- WO-A-94/03441
- THE JOURNAL OF ANTIBIOTICS, Bd. 46, Nr. 12, Dezember 1993 TOKYO, JAPAN, Seiten 1782-1787, KEIJI HASUMI ET AL. 'Lateritin, a new inhibitor of acyl-CoA: cholesterol acyltransferase produced by Giberella lateritium IFO 7188'
- CHEMICAL ABSTRACTS, vol. 78, no. 9, 5.März 1973 Columbus, Ohio, US; abstract no. 58777, SHEMYAKIN M.M. ET AL. 'Synthesis and antimicrobial activity of analogs of enniatin antibiotics.' & ZH. OBSHCH. KHIM., Bd. 42, Nr. 10, 1972 RUSS., Seiten 2320-2334,
- CHEMICAL ABSTRACTS, vol. 66, no. 18, 1.Mai 1967 Columbus, Ohio, US; abstract no. 80398, M.M. SHEMYAKIN ET AL. 'Depsipeptides. Mass spectrometric study of cyclodepsipeptides, 2,5-dioxomorpholines.' & IZV. AKAD. NAUK SSSR, SER. KHIM., Nr. 9, 1966 MOSCOW, Seiten 1539-1543,
- TETRAHEDRON LETTERS, Bd. 26, Nr. 43, 1985 OXFORD GB, Seiten 5257-5260, H.-G. LERCHEN ET AL. 'Stereoselektive Synthese von D-Alpha-Hydroxycarbonsäuren bzw. D-Alpha-Hydroxycarbonsäuren enthaltenden Depsipeptiden aus L-Aminosäuren.'

## Beschreibung

Die vorliegende Erfindung betrifft Dioxomorpholinen zur Verwendung bei der Bekämpfung von Endoparasiten, Dioxomorpholine und Verfahren zu ihrer Herstellung.

Bestimmte Dioxomorpholine und Verfahren zu ihrer Herstellung sind bereits bekannt (vgl. z.B. Liebigs Ann. Chem. 1952 (1982), Makromol. Chem., Rapid Commun., 6 (1985), 607; Tetrachedron, 37 (1981), 2797; WO 9403441A1;), über eine Verwendung dieser Verbindungen gegen Endoparasiten ist jedoch bisher nichts bekannt geworden.

Die vorliegende Erfindung betrifft:
1. Die Verwendung von Dioxomorpholinen der Formel (I) 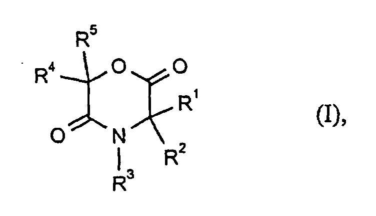 in welcher
   - R¹ und R²: unabhängig voneinander für Wasserstoff, geradkettiges oder verzweigtes Alkyl, Alkenyl, Cycloalkyl, Cycloalkylalkyl sowie für gegebenenfalls substituiertes Aryl, Arylalkyl, Heteroaryl, Heteroarylalkyl stehen, die gegebenenfalls substituiert sind,
   - R¹ und R²: gemeinsam für einen spirocyclischen Rest stehen, der gegebenenfalls substituiert ist,
   - R³: für Wasserstoff, geradkettiges oder verzweigtes Alkyl, Cycloalkyl, Aralkyl, Aryl, Heteroaryl, Heteroarylalkyl, die gegebenenfalls substituiert sind, steht,
   - R² und R³: gemeinsam mit den Atomen, an die sie gebunden sind, für einen 5- oder 6-gliedrigen Ring stehen, der gegebenenfalls substituiert sein kann,
   - R⁴ und R⁵: unabhängig voneinander für Wasserstoff, geradkettiges oder verzweigtes Alkyl, Alkenyl, Cycloalkyl, Cycloalkylalkyl sowie für gegebenenfalls substituiertes Aryl, Arylalkyl, Heteroaryl, Heteroarylalkyl stehen, die gegebenenfalls substituiert sind,
   - R⁴ und R⁵: gemeinsam für einen spirocyclischen Rest stehen, der gegebenenfalls substituiert ist
   sowie deren optische Isomere und Racemate,
   zur Herstellung von Mitteln zur Bekämpfung von Endoparasiten in der Medizin und Tiermedizin, wobei die gegebenenfalls substituierten Reste einen oder mehrere gleiche oder verschiedene Substituenten tragen, die ausgewählt sind aus der Gruppe: Alkyl mit 1 bis 4 Kohlenstoffatomen, Alkoxy mit 1 bis 4 Kohlenstoffatomen; Alkylthio mit 1 bis 4 Kohlenstoffatomen; Halogenalkyl mit 1 bis 4 Kohlenstoffatomen und 1 bis 5 gleichen oder verschiedenen Halogenatomen; Hydroxy; Halogen; Cyano; Nitro; Amino; Monoalkyl- und Dialkylamino mit 1 bis 4 Kohlenstoffatomen je Alkylgruppe; Alkylcarbonylamino mit 1 bis 4 Kohlenstoffatomen im Alkylteil; Benzoylamino, wobei Alkylcarbonylamino und Benzoylamino jeweils im Acylteil mit Halogen, Nitro, Trifluormethyl, Trifluormethoxy oder Trifluormethylthio substituiert sein können; Carboxyl; Carbalkoxy mit 2 bis 4 Kohlenstoffatomen, Alkylsulfinyl mit 1 bis 4 Kohlenstoffatomen, Halogenalkylsulfinyl mit 1 bis 4 Kohlenstoffatomen und 1 bis 5 Halogenatomen; Sulfonyl; Alkylsulfonyl mit 1 bis 4 Kohlenstoffatomen; Halogenalkylsulfonyl mit 1 bis 4 Kohlenstoffatomen und 1 bis 5 Halogenatomen; Arylsulfonyl mit 6 bis 10 Arylkohlenstoffatomen, Acyl, Aryl, Aryloxy, Heteroaryl, Heteroaryloxy, die ihrerseits einen der oben genannten Substituenten tragen können, sowie der Formiminorest -(CH)=N-O-Alkyl; und wobei Alkyl allein oder als Bestandteil eines Restes geradkettiges oder verzweigtes Alkyl mit 1 bis 9 Kohlenstoffatomen bedeutet; Alkenyl allein oder als Bestandteil eines Restes geradkettiges oder verzweigtes Alkenyl mit 2 bis 20 Kohlenstoffatomen bedeutet; Cycloalkyl mono-, di- und tricyclisches Cycloalkyl mit 3 bis 10 Kohlenstoffatomen bedeutet, Aryl Phenyl oder Naphthyl bedeutet, Arylalkyl 6 bis 10 Kohlenstoffatome im Arylteil und 1 bis 4 Kohlenstoffatome im Alkylteil, der geradkettig oder verzweigt sein kann, aufweist; Heteroaryl für einen 5- bis 7-gliedrigen Ring mit 1 bis 3 gleichen oder verschiedenen Heteroatomen ausgewählt aus Sauerstoff, Schwefel und Stickstoff steht.
   Bevorzugt verwendet werden Dioxomorpholinen der Formel (I) 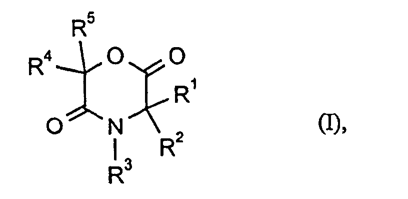 in welcher
   - R¹ und R²: unabhängig voneinander für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl stehen,
   - R¹ und R²: gemeinsam für einen spirocyclischen Rest stehen,
   - R³: für Wasserstoff, geradkettiges oder verzweigtes Alkyl, Cycloalkyl, Aralkyl, Aryl, Heteroaryl, Heteroarylalkyl, die gegebenenfalls substituiert sind, steht,
   - R² und R³: gemeinsam mit den Atomen, an die sie gebunden sind, für einen 5- oder 6-gliedrigen Ring stehen, der gegebenenfalls substituiert sein kann,
   - R⁴ und R⁵: unabhängig voneinander für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkyl aminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl stehen,
   - R⁴ und R⁵: gemeinsam für einen spirocyclischen Rest stehen,
   sowie deren optische Isomere und Racemate.
   Die Erfindung betrifft ferner:
2. Dioxomorpholine der Formel (I) 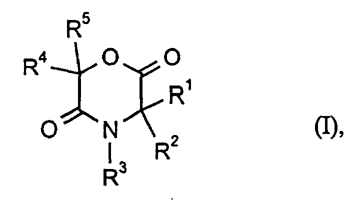 in welcher
   - R¹: für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl steht,
   - R²: für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl steht,
   - R¹ und R²: können zusammen für einen spirocyclischen Rest stehen,
   - R³: für geradkettiges oder verzweigtes Alkyl, Cycloalkyl, Aralkyl, Aryl, Heteroaryl, Heteroarylalkyl, die gegebenenfalls substituiert sind, steht,
   - R⁴: für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl steht,
   - R⁵: für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl steht,
   - R⁴ und R⁵: stehen gemeinsam für einen spirocyclischen Rest,
   mit Ausnahme solcher Verbindungen, in denen gleichzeitig
   a)
      - R¹: Wasserstoffbedeutet,
      - R²: Wasserstoff, Methyl, Benzyl oder Isopropyl bedeutet und
      - R³: Methyl oder alkylsubstituiertes Phenyl bedeutet,
      oder
   b)
      - R¹: Wasserstoff bedeutet,
      - R²: sec.-Butyl oder Isobutyl bedeutet,
      - R³: Methyl bedeutet,
      - R⁴: Wasserstoff bedeutet und
      - R⁵: Isopropyl bedeutet,
   wobei die gegebenenfalls substituierten Reste einen oder mehrere gleiche oder verschiedene Substituenten tragen, die ausgewählt sind aus der Gruppe: Alkyl mit 1 bis 4 Kohlenstoffatomen, Alkoxy mit 1 bis 4 Kohlenstoffatomen; Alkylthio mit 1 bis 4 Kohlenstoffatomen; Halogenalkyl mit 1 bis 4 Kohlenstoffatomen und 1 bis 5 gleichen oder verschiedenen Halogenatomen; Hydroxy; Halogen; Cyano; Nitro; Amino; Monoalkyl- und Dialkylamino mit 1 bis 4 Kohlenstoffatomen je Alkylgruppe; Alkylcarbonylamino mit 1 bis 4 Kohlenstoffatomen im Alkylteil; Benzoylamino, wobei Alkylcarbonylamino und Benzoylamino jeweils im Acylteil mit Halogen, Nitro, Trifluormethyl, Trifluormethoxy oder Trifluormethylthio substituiert sein können; Carboxyl; Carbalkoxy mit 2 bis 4 Kohlenstoffatomen, Alkylsulfinyl mit 1 bis 4 Kohlenstoffatomen, Halogenalkylsulfinyl mit 1 bis 4 Kohlenstoffatomen und 1 bis 5 Halogenatomen; Sulfonyl; Alkylsulfonyl mit 1 bis 4 Kohlenstoffatomen; Halogenalkylsulfonyl mit 1 bis 4 Kohlenstoffatomen und 1 bis 5 Halogenatomen; Arylsulfonyl mit 6 bis 10 Arylkohlenstoffatomen, Acyl, Aryl, Aryloxy, Heteroaryl, Heteroaryloxy, die ihrerseits einen der oben genannten Substituenten tragen können, sowie der Formiminorest -(CH)=N-O-Alkyl;
   und wobei Alkyl allein oder als Bestandteil eines Restes geradkettiges oder verzweigtes Alkyl mit 1 bis 9 Kohlenstoffatomen bedeutet; Alkenyl allein oder als Bestandteil eines Restes geradkettiges oder verzweigtes Alkenyl mit 2 bis 20 Kohlenstoffatomen bedeutet; Cycloalkyl mono-, di- und tricyclisches Cycloalkyl mit 3 bis 10 Kohlenstoffatomen bedeutet, Aryl Phenyl oder Naphthyl bedeutet, Arylalkyl 6 bis 10 Kohlenstoffatome im Arylteil und 1 bis 4 Kohlenstoffatome im Alkylteil, der geradkettig oder verzweigt sein kann, aufweist; Heteroaryl für einen 5- bis 7-gliedrigen Ring mit 1 bis 3 gleichen oder verschiedenen Heteroatomen ausgewählt aus Sauerstoff, Schwefel und Stickstoff steht;
   sowie deren optische Isomere und Racemate.
3. Verfahren zur Herstellung der Dioxomorpholine der Formel (I) gemäß Punkt 2 (oben) 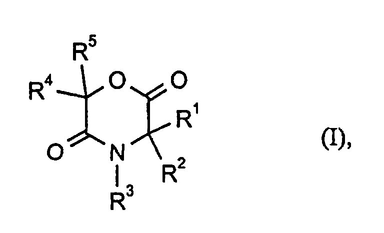 in welcher
   die Reste R¹ bis R⁵ die unter Punkt 2 angegebene Bedeutung haben,
   dadurch gekennzeichnet, daß man
   a) offenkettige Depsipeptide der Formel (II) 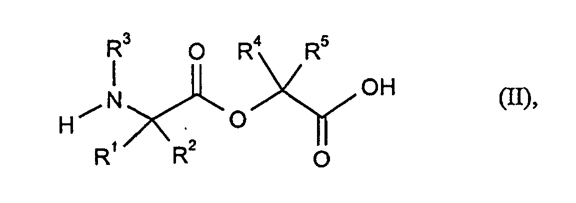 in welcher
      die Reste R¹ bis R⁵ die oben angegebene Bedeutung haben,
      in Gegenwart eines Verdünnungsmittels und in Gegenwart eines Kopplungsreagenzes cyclisiert,
      oder
   b) Verbindungen der Formel (I) 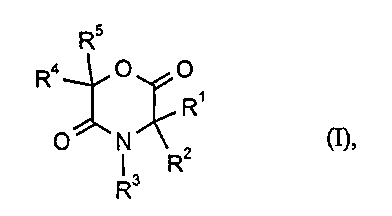 in welcher
      - R³: für Wasserstoff steht, und
      R¹, R², R⁴ und R⁵ die oben angegebene Bedeutung haben
      mit Verbindungen der Formel (III)

      X-R³ (III),

      in welcher
      - R³: für geradkettiges oder verzweigtes Alkyl, Cycloalkyl, Aralkyl, Heteroarylalkyl, die gegebenenfalls substituiert sein können, steht,
      - X: für I, Cl oder Br steht,
   in Gegenwart eines Verdünnungsmittels und einer Base umsetzt.
4. Offenkettige Depsipeptide der Formel (II) 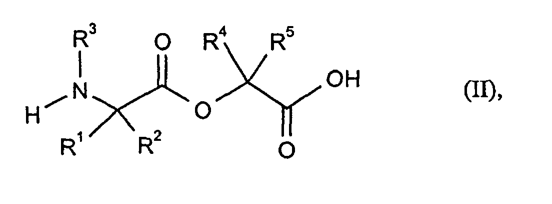 in welcher
   R¹ bis R⁶ unter Punkt 2 angegebene Bedeutungen haben.
5. Verfahren zur Herstellung der offenkettigen Depsipeptide der Formel (II) 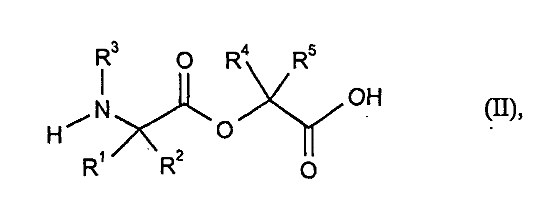 in welcher
   R¹ bis R⁵ die oben (unter Punkt 2) angegebene Bedeutung besitzen,
   dadurch gekennzeichnet, daß man
   a) Verbindungen der Formel (IV) 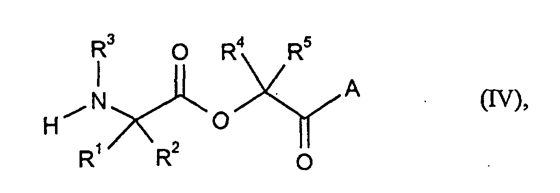 in welcher
      - A: für tert.-Butoxy steht,
      R¹ bis R⁵ die oben angegebenen Bedeutungen besitzen,
      in Gegenwart eines Verdünnungsmittels und einer Protonensäure verseift, oder
   b) Verbindungen der Formel (V) 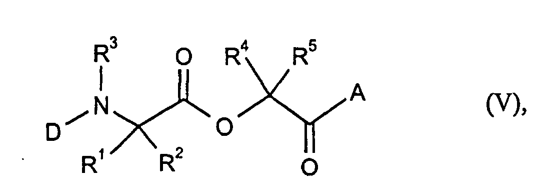 in welcher
      - A: für tert.-Butoxy steht,
      - D: für tert.-Butoxycarbonyl (-CO₂^{t}Bu) steht,
      R¹ bis R⁵ die oben angegebenen Bedeutungen haben,
   in Gegenwart eines Verdünnungsmittels und einer Protonensäure verseift.
6. Verbindungen der Formel (IV) 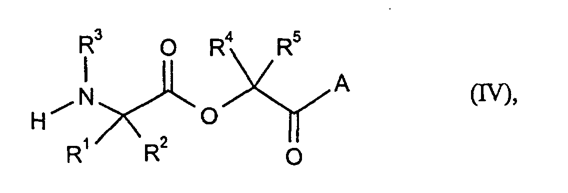 in welcher
   - A: für tert.-Butoxy steht,
   R¹ bis R⁵ die oben angegebenen Bedeutungen haben,
   mit der Maßgabe, dass wenn
   R¹ und R⁴ für Wasserstoff stehen.
   - R²: nicht Wasserstoff, geradkettiges oder verzweigtes Alkyl, Alkenyl, Cycloalkyl, Aryl, Arylalkyl oder Heteroarylalkyl, die jeweils gegebenenfalls substituiert sind, bedeutet.
7. Verfahren zur Herstellung von Verbindungen der Formel (IV) 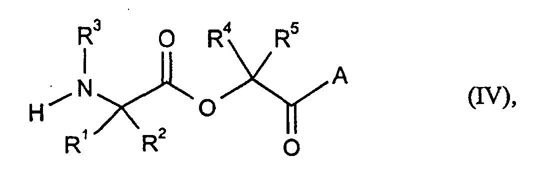 in welcher
   - A: für tert.-Butoxy steht,
   R¹ bis R⁵ die oben angegebenen Bedeutungen haben,
   dadurch gekennzeichnet, daß man Verbindungen der Formel (VI) 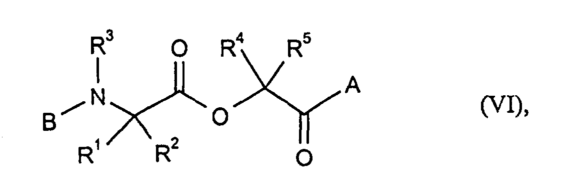 in welcher
   - A: für tert.-Butoxy,
   - B: für Benzyl,
   R¹ bis R⁵ die oben angegebene Bedeutung haben,
   in Gegenwart eines Verdünnungsmittels und eines Katalysators hydrogenolisiert.
8. Verbindungen der Formel (V) 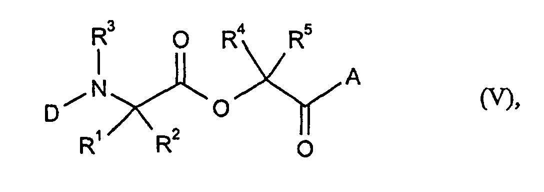 in welcher
   - A: für tert.-Butoxy steht,
   - D: für tert.-Butoxycarbonyl steht,
   R¹ bis R⁵ die oben angegebenen Bedeutungen haben.
9. Verfahren zur Herstellung von Verbindungen der Formel (V) 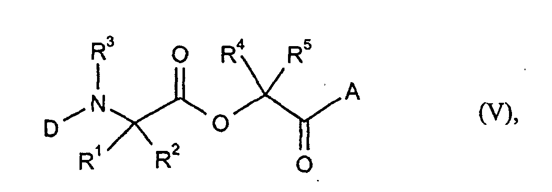 in welcher
   - A: für tert.-Butoxy steht,
   - D: für tert.-Butoxycarbonyl steht,
   R¹ bis R⁵ die oben angegebenen Bedeutungen besitzen,
   dadurch gekennzeichnet, daß man Verbindungen der Formel (VII) 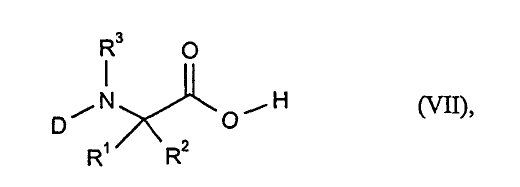 in welcher
   - D: für tert.-Butoxycarbonyl steht,
   R¹ bis R³ die oben angegebene Bedeutung besitzen, in Form ihres Alkalimetallsalzes, bevorzugt ihres Caesiumsalzes und eine α-Halogencarbonsäure der Formel (VIII) 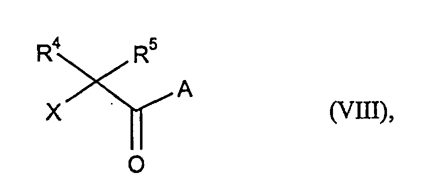 in welcher
   - X: für Cl oder Br und
   - A: für tert.-Butoxy steht,
   R⁴ und R⁵ die oben angegebene Bedeutung haben,
   in Gegenwart eines Verdünnungsmittels umsetzt.
10. Verbindungen der Formel (VI) 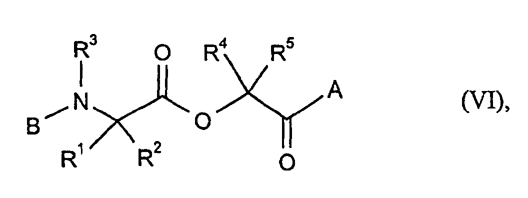 in welcher
   - A: für tert.-Butoxy,
   - B: für Benzyl steht,
   R¹ bis R⁵ die oben angegebene Bedeutung besitzen,
   mit der Maßgabe, dass wenn
   - R¹ und R⁴: für Wasserstoff stehen,
   - R²: nicht Wasserstoff, geradkettiges oder verzweigtes Alkyl, Alkenyl, Cycloalkyl, Aryl, Arylalkyl oder Heteroarylalkyl, die jeweils gegebenenfalls substituiert sind, bedeutet.
11. Verfahren zur Herstellung von Verbindungen der Formel (VI) 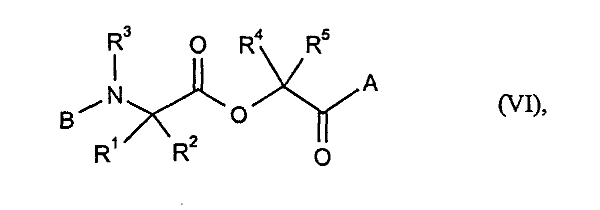 in welcher
   - A: für tert.-Butoxy steht,
   - B: für Benzyl sowie
   R¹ bis R⁵ die oben angegebene Bedeutung haben,
   dadurch gekennzeichnet, daß man Verbindungen der Formel (IX) 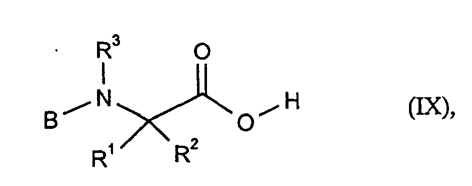 in welcher
   - B: für Benzyl steht,
   R¹ bis R³ die oben angegebene Bedeutung haben, in Form ihres Alkalimetallsalzes, bevorzugt ihres Caesiumsalzes,
   und eine α-Halogencarbonsäure der Formel (VIII) 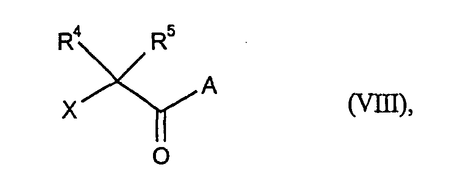 in welcher
   - X: für Cl, Br steht,
   - A: für tert.-Butoxy,
   R⁴ und R⁵ die oben angegebene Bedeutung besitzen
   in Gegenwart eines Verdünnungsmittels umsetzt.

Für den Fall, daß R⁵ für Benzyl steht, kann der Phenylring durch übliche Substitutionsreaktionen derivatisiert werden.

Als Beispiel für eine solche Substitutionsreaktion sei die folgende beschriebene Nitrierung genannt.

In den folgenden Formeln haben die Substituenten R¹ bis R⁴ die unter Punkt 1 (oben) angegebene Bedeutung.

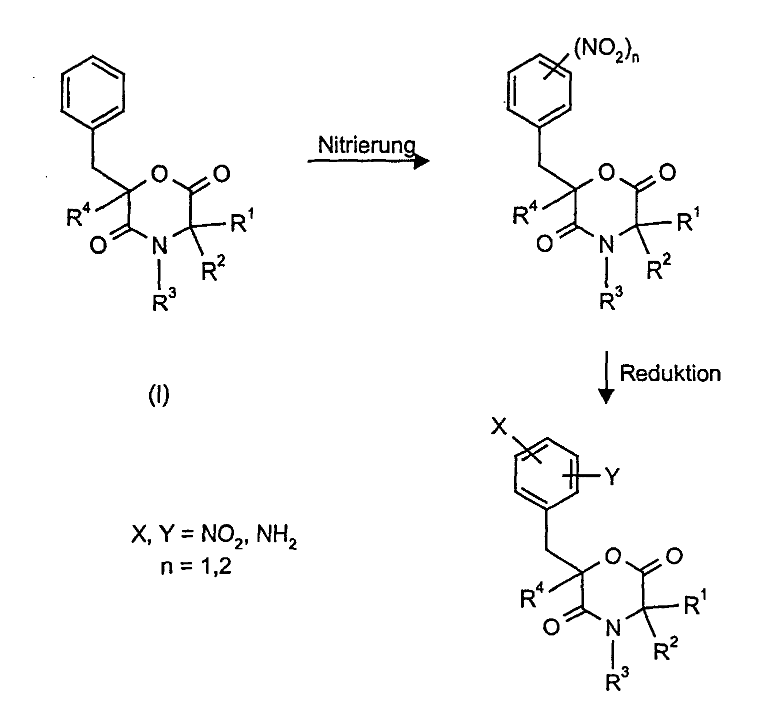

Die Nitrogruppe (I) kann reduziert werden zu mono- bzw. bisaminosubstituierten Phenylderivaten. Diese können in Folgereaktionen acyliert bzw. alkyliert werden, z.B.

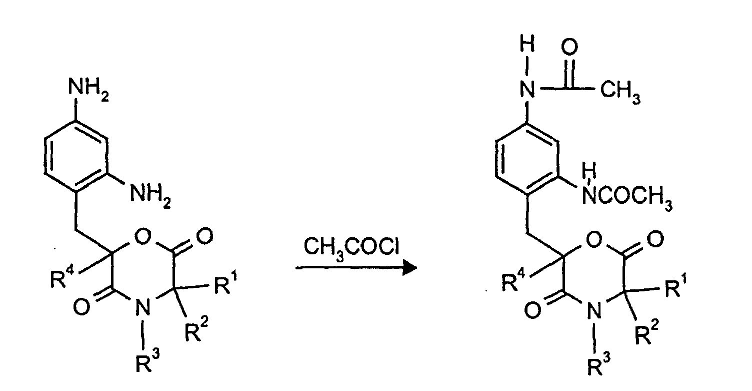

Die Dioxomorpholine der Formel (I) sowie deren Säureadditions-Salze und Metallsalz-Komplexe besitzen sehr gute endoparasitizide, insbesondere anthelmintische Wirkung und können bevorzugt im Bereich der Veterinärmedizin eingesetzt werden. Überraschenderweise zeigen die erfindungsgemäßen Stoffe bei der Bekämpfung von Wurmerkrankungen eine deutlich bessere Wirksamkeit als konstitutionell ähnliche, vorbekannte Verbindungen gleicher Wirkungsrichtung.

Gegebenenfalls substituiertes Alkyl allein oder als Bestandteil eines Restes in den allgemeinen Formeln bedeutet geradkettiges oder verzweigtes Alkyl mit 1 bis 9, insbesondere 1 bis 5 Kohlenstoffatomen. Beispielhaft und vorzugsweise seien gegebenenfalls substituiertes Methyl, Ethyl, n- und i-Propyl, n-, i- und t-Butyl, genannt.

Gegebenenfalls substituiertes Alkenyl allein oder als Bestandteil eines Restes in den allgemeinen Formeln bedeutet geradkettiges oder verzweigtes Alkenyl mit 2 bis 20, insbesondere 2 bis 18 Kohlenstoffatomen. Beispielhaft und vorzugsweise seien gegebenenfalls substituiertes Ethenyl, Propenyl-(1), Propenyl-(2) und Butenyl-(3) genannt.

Gegebenenfalls substituiertes Cycloalkyl in den allgemeinen Formeln bedeutet mono-, bi- und tricyclisches Cycloalkyl mit 3 bis 10, insbesondere 3, 5 oder 6 Kohlenstoffatomen. Beispielsweise und vorzugsweise seien gegebenenfalls substituiertes Cyclopropyl, Cyclobutyl, Cyclopentyl, Cyclohexyl, Cycloheptyl, Bicyclo[2.2.1]heptyl, Bicyclo[2.2.2]octyl und Adamantyl genannt.

Gegebenenfalls substituiertes Alkoxy in den allgemeinen Formeln bedeutet geradkettiges oder verzweigtes Alkoxy mit 1 bis 6, insbesondere 1 bis 4 Kohlenstoffatomen. Beispielhaft und vorzugsweise seien gegebenenfalls substituiertes Methoxy, Ethoxy, n- und i-Propoxy und n-, o- und t-Butoxy genannt.

Gegebenenfalls substituiertes Alkylthio in den allgemeinen Formeln bedeutet geradkettiges oder verzweigtes Alkylthio mit 1 bis 6, insbesondere 1 bis 4 Kohlenstoffatomen. Beispielhaft und vorzugsweise seien gegebenenfalls substituiertes Methylthio, Ethylthio, n- und i-Propylthio, n-, o- und t-Butylthio genannt.

Halogenalkyl in den allgemeinen Formeln enthält 1 bis 4, insbesondere 1 oder 2 Kohlenstoffatome und 1 bis 5 gleiche oder verschiedene Halogenatome, wobei als Halogenatome vorzugsweise Fluor, Chlor und Brom, insbesondere Fluor und Chlor stehen. Beispielhaft seien Trifluormethyl, Chlordi-fluormethyl, Brommethyl, 2,2,2-Trifluorethyl und Pentafluorethyl, Perfluor-t-butyl genannt.

Gegebenenfalls substituiertes Aryl in den allgemeinen Formeln bedeutet gegebenenfalls substituiertes Phenyl oder Naphthyl, insbesondere Phenyl.

Gegebenenfalls substituiertes Arylalkyl in den allgemeinen Formeln bedeutet gegebenenfalls im Arylteil und/oder Alkylteil substituiertes Aralkyl mit 6 oder 10, insbesondere 8 Kohlenstoffatomen im Arylteil (vorzugsweise Phenyl oder Naphthyl, insbesondere Phenyl) und 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Gegebenenfalls substituierte Heteroaryl allein oder als Bestandteil eines Restes bedeutet in den allgemeinen Formeln 5- bis 7-gliedrige Ringe mit 1 bis 3, insbesondere 1 oder 2 gleichen oder verschiedenen Heteroatomen. Als Heteroatome stehen Sauerstoff, Schwefel oder Stickstoff. Beispielhaft und vorzugsweise seien gegebenenfalls substituiertes Furyl, Thienyl, Pyrazolyl, Imidazolyl, 1,2,3- und 1,2,4-Triazolyl, Oxazolyl, Isoxazolyl, Thiazolyl, Isothiazolyl, 1,2,3-, 1,3,4-, 1,2,4- und 1,2,5-Oxadiazolyl, Azepinyl, Pyrrolyl, Isopyrrolyl, Pyridyl, Piperazinyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl und 1,2,4-Diazepinyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butyltio; Halogenalkyl mit vorzugsweise 1 bis 4, insbesondere 1 oder 2 Kohlenstoffatomen und 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Difluormethyl, Trifluormethyl; Hydroxy; Halogen, vorzugsweise Fluor, Chlor, Brom und Iod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-Butylamino. Acetylierte Aminoreste wie C₁₋₄ Alkylcarbonylamino, Benzoylamino die im Acylteil substituiert mit Halogen, Nitro, Trifluormethyl, Trifluormethoxy, Trifluormethylthio sein können. Acylreste wie Carboxyl; Carbalkoxy mit vorzugsweise 2 bis 4, insbesondere 2 oder 3 Kohlenstoffatomen, wie Carbomethoxy und Carboethoxy; Alkylsulfinyl mit 1 bis 4, insbesondere 1 bis 2 Kohlenstoffatomen, Halogenalkylsulfinyl mit 1 bis 4, insbesondere 1 bis 2 Kohlenstoffatomen und 1 bis 5 Halogenatomen wie Trifluormethylsulfinyl; Sulfonyl (-SO₃H); Alkylsulfonyl mit vorzugsweise 1 bis 4, insbesondere 1 oder 2 Kohlenstoffatomen, wie Methylsulfonyl und Ethylsulfonyl; Halogenalkylsulfonyl mit 1 bis 4, insbesondere 1 bis 2 Kohlenstoffatomen und 1 bis 5 Halogenatomen wie Trifluormethylsulfonyl, Perfluor-t,n,s-butylsulfonyl; Arylsulfonyl mit vorzugsweise 6 oder 10 Arylkohlenstoffatomen, wie Phenylsulfonyl; Acyl, Aryl, Aryloxy, Heteroaryl, Heteroaryloxy, die ihrerseits einen der oben genannten Substituenten tragen können sowie der Formininorest

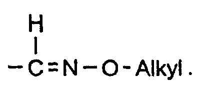

Bevorzugt sind Verbindungen der Formel ( I ),
in welcher
- R³: für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl(Fmoc)amino-C₁-C₆-alkyl, insbesondere 9-Fluor enyl-methoxycarbonyl(Fmoc)aminopropyl, 9-Fluorenylmethoxycarbonyl(Fmoc)aminobutyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl, die gegebenenfalls durch Reste aus der Reihe Halogen, insbesondere Fluor, Chlor Brom oder Iod, Hydroxy, Nitro, CN, NH₂, C₁-C₄-Alkoxy, insbesondere Methoxy oder Ethoxy, C₁-C₄-Alkyl, insbesondere Methyl, substituiert sein können,
- R¹, R², R⁴ und R⁵: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl, Thienylmethyl, Thiazolylmethyl, Pyridylmethyl, die gegebenenfalls durch Reste aus der Reihe Halogen, insbesondere Fluor, Chlor, Brom oder Iod, Hydroxy, Sulfonyl (SO₃H), CN, NO₂, Amino, Di(C₁-C₄-alkyl)-amino z.B. Dimethylamino, acetylierte Amino zum Beispiel Acetylamino, Benzoylamino die gegebenenfalls im Acylteil durch einen oder mehrere gleiche oder verschiedene der oben angegebenen Reste substituiert sein können, C₁-C₄-Alkoxy, insbesondere Methoxy oder Ethoxy, C₁-C₄-Alkyl, insbesondere Methyl, substituiert sein können,
sowie deren optische Isomere und Racemate,
mit Ausnahme solcher Verbindungen, in denen gleichzeitig
a)
   - R¹: Wasserstoff bedeutet,
   - R²: Wasserstoff, Methyl, Benzyl oder Isopropyl bedeutet und
   - R³: Methyl oder alkylsubstituiertes Phenyl bedeutet,
   oder
b)
   - R¹: Wasserstoff bedeutet,
   - R²: sec.-Butyl oder Isobutyl bedeutet,
   - R³: Methyl bedeutet,
   - R⁴: Wasserstoff bedeutet und
   - R⁵: Isopropyl bedeutet.

Besonders bevorzugt sind Verbindungen der Formel (I),
in welcher
- R³: für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-AlkoxycarbonylaminoC₁-C₆-alkyl, insbesondere tert.-Butoxycarbonylaminopropyl, tert.-Butoxycarbonylaminobutyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl, die gegebenenfalls durch einen oder mehrere gleiche oder verschiedene der oben angegebenen Reste substituiert sein können,
- R¹, R², R⁴ und R⁵: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, Hydroxy-C₁-C₆-alkyl, insbesondere Hydroxymethyl, Aryl-C₁-C₄-alkyloxy-C₁-C₆-alkyl, insbesondere Benzyloxymethyl, 1-Benzyloxyethyl, C₁-C₄-Alkoxycarbonyl-C₁-C₆-alkyl, insbesondere Methoxycarbonylmethyl, Ethoxycarbonylethyl, C₁-C₄-Aryl-alkoxycarbonyl-C₁-C₆-alkyl, insbesondere Benzyloxycarbonylmethyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl, Thienylmethyl, die gegebenenfalls durch einen oder mehrere gleiche oder verschiedene der oben angegebenen Reste substituiert sein können,
sowie deren optische Isomere und Racemate,
mit Ausnahme solcher Verbindungen, in denen gleichzeitig
a)
   - R¹: Wasserstoff bedeutet,
   - R²: Wasserstoff, Methyl, Benzyl oder Isopropyl bedeutet und
   - R³: Methyl oder alkylsubstituiertes Phenyl bedeutet,
   oder
b)
   - R¹: Wasserstoff bedeutet,
   - R²: sec.-Butyl oder Isobutyl bedeutet,
   - R³: Methyl bedeutet,
   - R⁴: Wasserstoff bedeutet und
   - R⁵: Isopropyl bedeutet.

Ganz besonders bevorzugt sind Verbindungen der Formel ( I ),
in welcher
- R³: für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, C₃-C₇-Cycloalkyl-C₁-C₄-alkyl, insbesondere Cyclohexylmethyl, Phenyl-C₁-C₄-alkyl, insbesondere Phenylmethyl stehen,
- R¹, R², R⁴ und R⁵: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, C₂-C₈- Alkenyl, insbesondere Vinyl, Allyl, C₃-C₇-Cycloalkyl-C₁-C₄-alkyl, insbesondere Cyclohexylmethyl, Phenyl-C₁-C₄-alkyl, insbesondere Phenylmethyl, Thienylmethyl, die gegebenenfalls durch einen oder mehrere gleiche oder verschiedene der oben angegebenen Reste substituiert sein können,
sowie deren optische Isomere und Racemate,
mit Ausnahme solcher Verbindungen, in denen gleichzeitig
a)
   - R¹: Wasserstoff bedeutet,
   - R²: Wasserstoff, Methyl, Benzyl oder Isopropyl bedeutet und
   - R³: Methyl oder alkylsubstituiertes Phenyl bedeutet,
   oder
b)
   - R¹: Wasserstoff bedeutet,
   - R²: sec.-Butyl oder Isobutyl bedeutet,
   - R³: Methyl bedeutet,
   - R⁴: Wasserstoff bedeutet und
   - R⁵: Isopropyl bedeutet,.

Die Verbindungen der allgemeinen Formel (I), können in optisch aktiven, stereoisomeren Formen oder als racemische Gemische vorliegen und verwendet werden. Vorzugsweise werden die optisch aktiven, stereoisomeren Formen der Verbindungen der allgemeinen Formel (I) verwendet.

Im einzelnen seien folgende Verbindungen der allgemeinen Formel ( I ) genannt, in welcher die Reste R¹ bis R⁵ die folgende Bedeutung haben:

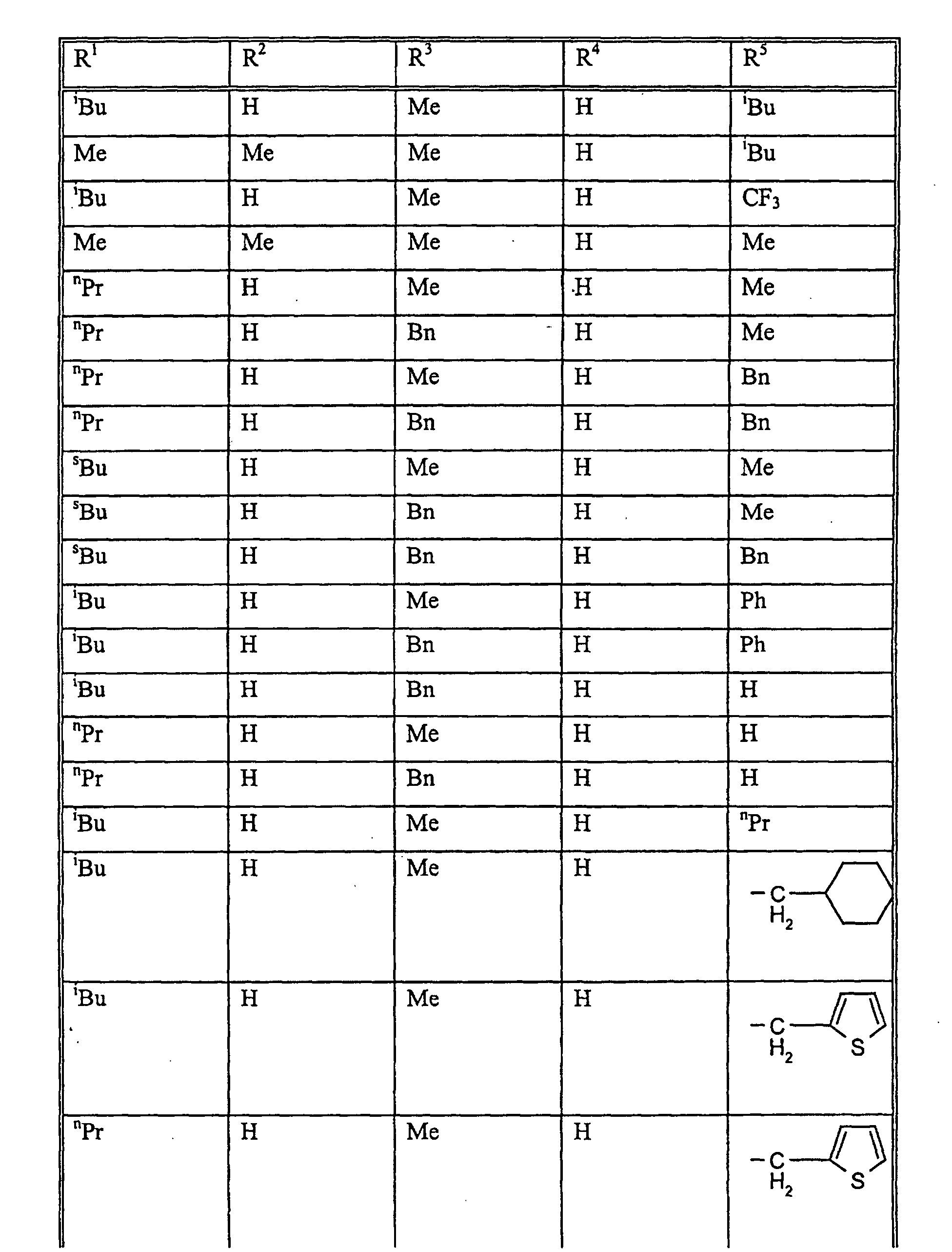

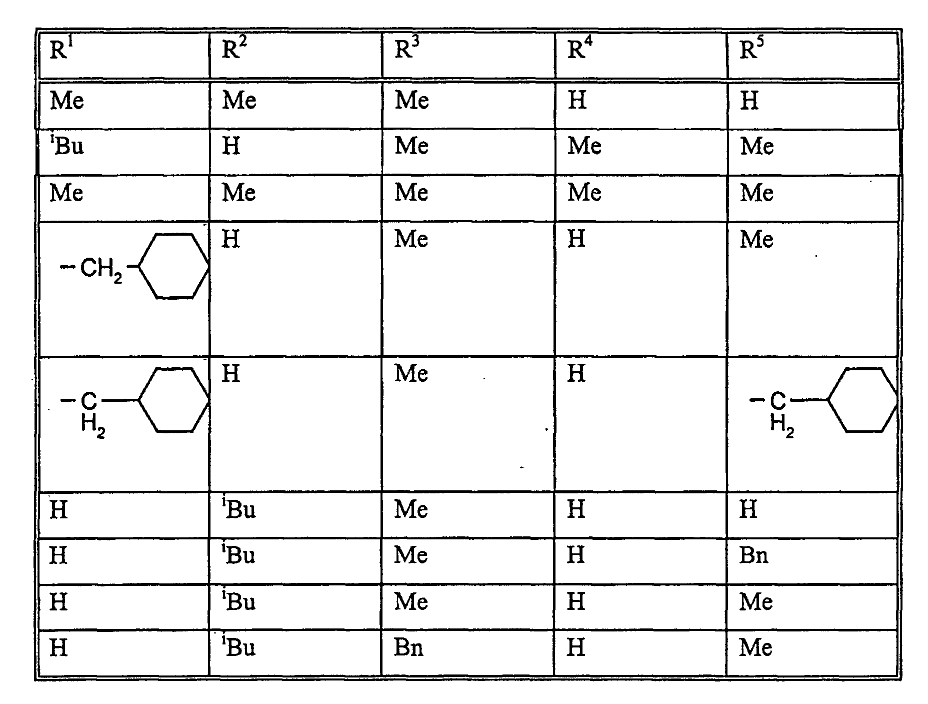

In dieser und den folgenden Tabelle steht jeweils
Bu für Butyl
Me für Methyl
Bn für Benzyl
Pr für Propyl
Ph für Phenyl

Von den neuen Verbindungen der allgemeinen Formel (I) sind diejenigen bevorzugt und besonders bevorzugt in denen die Substituenten die oben angegebenen bevorzugten Definitionen besitzen.

Die Verbindungen der allgemeinen Formel (I) sind teilweise bekannt (s. oben) oder können nach den dort angegebenen Verfahren erhalten werden.

Die neuen Verbindungen der Formel (I) lassen sich nach dem von U. Schmidt et al. für makrocyclische Peptidalkaloide angewendeten Verfahren (vgl. z.B.: U. Schmidt et al. in Synthesis (1991) S. 294-300 [Didemnin A, B und C]; Angew. Chem. 96 (1984) S. 723-724 [Dolastatin 3]; Angew. Chem. 102 (1990) S. 562-563 [Fenestin A]; Angew. Chem. 97 (1985) S. 606-607 [Ulicyclamid]; J. Org. Chem. 47 (1982) S. 3261-3264) herstellen.

Die neuen Verbindungen der allgemeinen Formel (I) lassen sich nach den oben unter Punkt 3 angegebenen Verfahren herstellen.

Setzt man bei Verfahren 3 zur Herstellung der neuen Dioxomorpholine (I) als Verbindungen der Formel (II) N-Methyl-L-leucyl-D-milchsäure ein, so läßt sich das Verfahren durch folgendes Reaktionsschema wiedergeben:

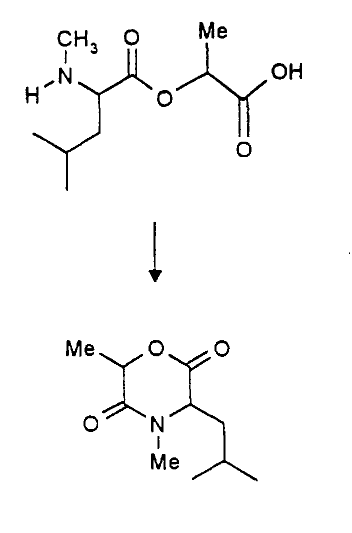

Die zur Durchführung des Verfahrens 3a als Ausgangsstoffe benötigten offenkettigen Didepsipeptide sind durch die Formel (II) allgemein definiert. In dieser Formel stehen R¹ bis R⁵ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten Didepsipeptide der Formel (II) sind neu. Ihre Herstellung wird weiter unten beschrieben.

Im einzelnen seien folgende Verbindungen der allgemeinen Formel (II) genannt, in welcher die Reste R¹ bis R⁵ die folgende Bedeutung haben:

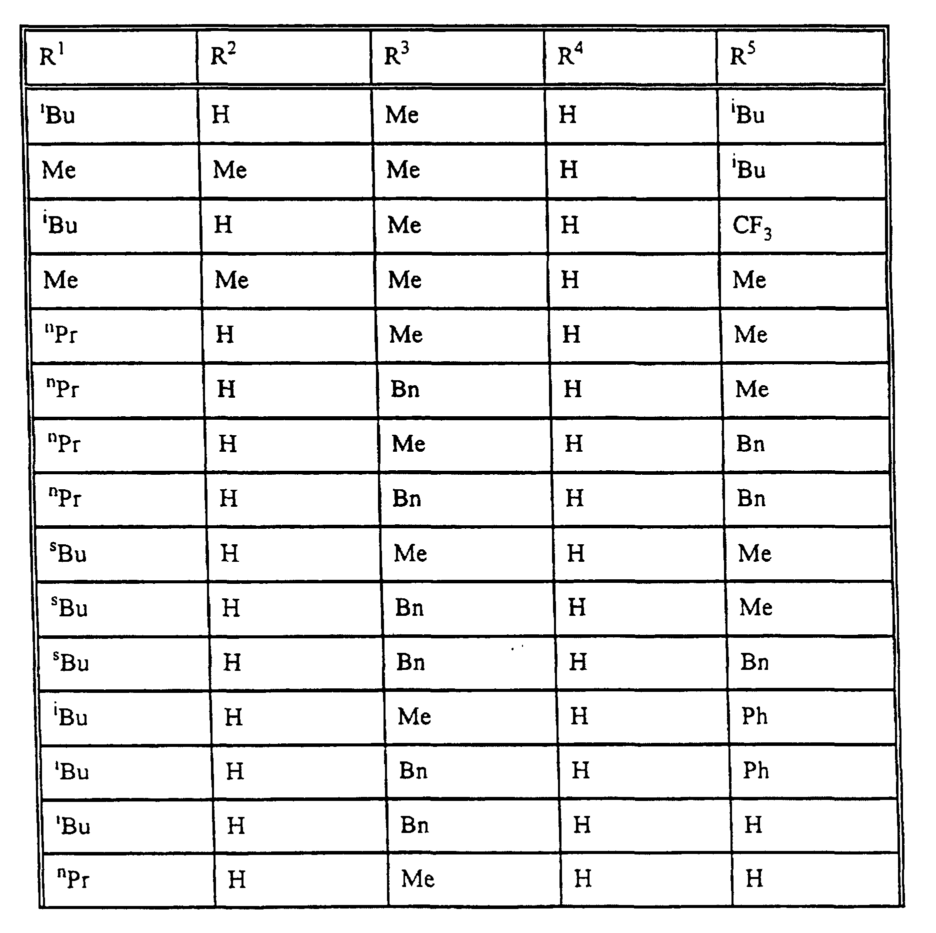

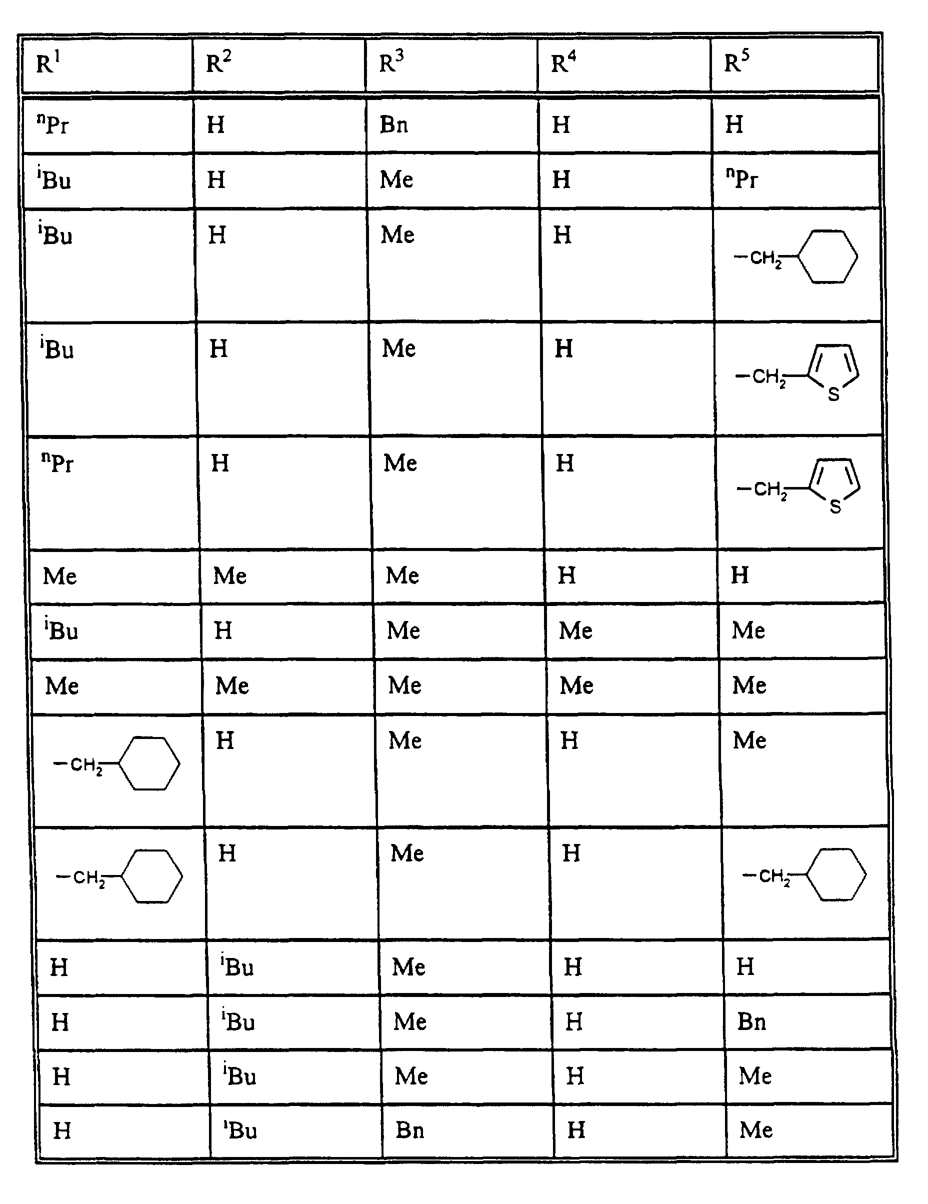

Bei Verfahren 3a werden Tetradepsipeptide in Gegenwart von Verdünnungsmitteln und geeigneten Kopplungsreagenzien cyclisiert.

Als Kopplungsreagenzien eignen sich alle Verbindungen die zur Knüpfung einer Aminbindung geeignet sind (vgl. z.B.: Houben-Weyl, Methoden der Organischen Chemie, Band 15/2; Bodenzky et al., Peptide Synthesis 2nd ed., Wiley u. Sons, New York 1976).

Vorzugsweise kommen folgende Methoden in Frage: Aktivestermethode mit Pentafluorphenol (PfP), N-Hydroxysuccinimid, 1-Hydroxybenzotriazol, Kopplung mit Carbodiimiden wie Dicyclohexylcarbodiimid oder N'-(3-Dimethylaminopropyl)-N-ethylcarbodiimid (EDC) sowie die Gemischte-Anhydrid-Methode oder die Kopplung mit Phosphoniumreagenzien, wie Benzotriazol-1-yl-oxy-tris(dimethylaminophosphonium)-hexafluorphosphat (BOP), Bis-(2-oxo-3-oxazolidinyl)-phosphoniumsäurechlorid (BOP-Cl) oder mit Phosphonsäureesterreagenzien wie Cyanphosphoniumsäurediethylester (DEPC) und Diphenylphosphorylazid (DPPA).

Besonders bevorzugt ist die Kopplung mit Bis(2-oxo-3-oxazolidinyl)-phosphoniumsäurechlorid (BOP-Cl) und N'-(3-Dimethylaminopropyl)-N-ethylcarbodiimid (EDC) in Gegenwart von 1-Hydroxybenzotriazol (HOBt).

Die Umsetzung erfolgt bei Temperaturen von 0 bis 150°C, bevorzugt bei 20 bis 100°C, besonders bevorzugt bei Raumtemperatur.

Als Verdünnungsmittel kommen alle inerten organischen Lösungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethylund Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile wie z.B. Acetonitril und Propionitril, Benzonitril, Glutarsäuredinitril, darüber hinaus Amide wie z.B. Dimethylformamid, Dimethylacetamid und N-Methylpyrrolidon, sowie Dimethylsulfoxid, Tetramethylensulfon und Hexamethylphosphorsäuretriamid.

Die Cyclisierung wird in Gegenwart einer Base durchgeführt.

Als Basen kommen anorganische und organische Basen in Frage. Als Basen seien genannt: Alkali- und Erdalkalihydroxide, -carbonate, -hydrogencarbonate, -alkoholate, ferner Amine wie insbesondere tertiäre Amine, z.B. Trimethylamin, Triethylamin, N-Methylmorpholin, Pyridin, Picoline, N-Ethylpyrrolidin, Diazabicyclo(4,3,0)-undecen (DBU), 1,4-Diazabicyclo(2,2,2)octan (DABCO), Diazabicyclo(3,2,0)nonen (DBN), Ethyl-diisopropylamin.

Die Verbindungen der Formeln (II) und die Basen werden im Verhältnis 1:1 bis 1:2 zueinander eingesetzt. Bevorzugt ist ein etwa äquimolares Verhältnis.

Nach erfolgter Umsetzung wird das Verdünnungsmittel abdestilliert und die Verbindungen der Formel (I) in üblicher Weise, z.B. chromatographisch, gereinigt.

Die als Ausgangsverbindungen verwendeten, Didepsipeptide der Formel (II) können nach an sich bekannten Verfahren hergestellt werden, beispielsweise wie es von H.-G. Lerchen und H. Kunz (Tetrahedron Lett. 26 (43) (1985) S. 5257-5260; 28 (17) (1987) S. 1873-1876) unter Ausnutzung der Veresterungsmethode nach B. F. Gisin (Helv. Chim. Acta 56 (1973) S. 1476) beschrieben ist.

Die als Ausgangsmaterialien verwendeten Aminosäuren und 2-Halogen-carbonsäurederivate sind teilweise bekannt (vgl. z.B.: N-Methyl-aminosäuren: R Bowmann et al. J Chem. Soc. (1950) S. 1346; J. R. McDermott et al. Can. J. Chem. 51 (1973) S. 1915; H. Wurziger et al., Kontakte (Merck.Darmstadt) 3 (1987) S. 8; 2-Halogencarbonsäurederivate: S. M. Birnbaum et al. J. Amer. Chem. Soc. 76 (1954) S. 6054, C. S. Rondestvedt, Jr. et al. Org. Reactions 11 (1960) S. 189 [Review]) bzw. können nach den dort beschriebenen Verfahren erhalten werden.

Die offenkettigen Didepsipeptide der Formel (II) können nach einem Verfahren erhalten werden werden, das die folgenden aufeinander folgenden Stufen umfaßt
a) Synthese der Didepsipeptide der Formeln (V) und (VI) nach Verfahren 9 und 11. 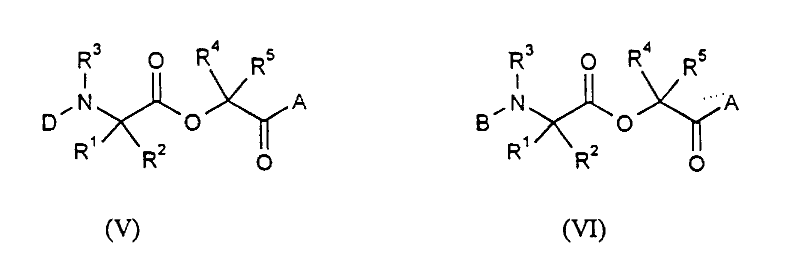
worin B eine N-terminale Schutzgruppe, wie z.B. die Benzyl oder Benzyloxycarbonylgruppe oder D für tert.-Butyloxycarbonyl und A eine C-terminale Schutzgruppe, wie z.B. die tert-Butoxygruppe, darstellt.

Dies entspricht für Formel (VI) beispielsweise dem folgenden Reaktionsschema:

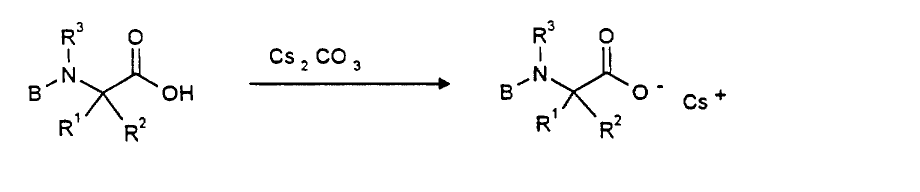

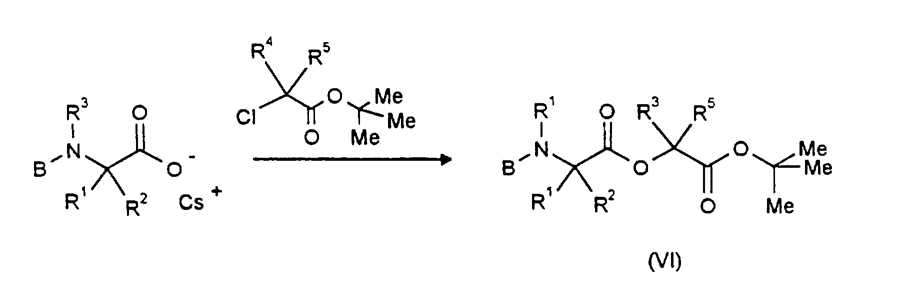

Die Herstellung der erfindungsgemäßen, enantiomerenreinen Verbindungen der Formeln (V) und (VI) kann gegebenenfalls auch über die Trennung der Diastereomere nach üblichen Methoden wie beispielsweise Kristallisation, durch Säulenchromatographie oder durch Craig-Verteilung erfolgen. Welches das optimale Verfahren ist, muß von Fall zu Fall entschieden werden, manchmal ist es auch zweckmäßig, Kombinationen der einzelnen Verfahren zu benutzen.

Am Ende dieser Stufe kann eine Entfernung der N-terminalen Schutzgruppe aus den Verbindungen der Formel (VI) in an sich bekannter Weise durchgeführt werden, beispielsweise durch katalytische Hydrierung, zur Herstellung der Derivate der Formel (IV). In an sich bekannter Weise kann eine Abspaltung der C-terminalen Schutzgruppe aus den Derivaten der Formel (IV) zur Darstellung der Verbindungen der Formel (II) erfolgen.

Verbindungen der Formel (II) kann auch erhalten werden, indem eine gleichzeitige Abspaltung der N- und C-terminalen Schutzgruppen aus den Derivaten der Formel (V) erfolgt.

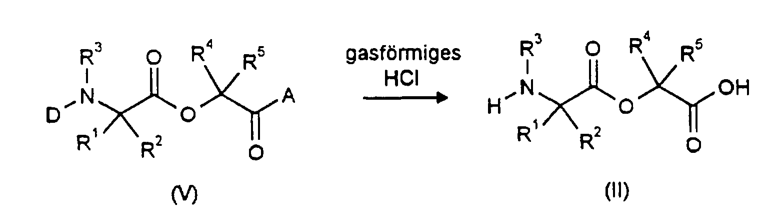

Die Abspaltung der N-terminalen Schutzgruppen durch Hydrogenolyse in den Verfahren 7 wird besonders bevorzugt mit Hydrierungsmitteln wie Wasserstoff in Gegenwart der üblichen Hydrierungskatalysatoren, wie z.B. Raney-Nickel, Palladium und Platin, durchgeführt.

Das Verfahren wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Methyl-tert.-butylether, Glykoldimethylether und Diglykoldimethylether, Tetrahydrofuran und Dioxan, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid; ferner auch Alkohole wie Methanol, Ethanol, Propanol, Isopropanol, Butanol, Isobutanol, sec.-Butanol, tert.-Butanol, Pentanol, Isopentanol, sec.-Pentanol und tert.-Pentanol sowie auch Wasser.

Die Reaktionstemperaturen können bei dem erfindungsgemäßen Verfahren in einem größeren Bereich variiert werden Im allgemeinen arbeitet man bei Temperaturen zwischen -20°C und +200°C, vorzugsweise bei Temperaturen zwischen 0°C und 120°C.

Das Verfahren wird im allgemeinen unter Normaldruck durchgeführt. Es ist jedoch auch möglich, unter erhöhtem, im allgemeinen zwischen 10 und 100 bar, zu arbeiten.

Die Abspaltung der C-terminalen Schutzgruppen durch Verseifung in den Verfahren 5a) und 5b) wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Die Reaktion wird in Gegenwart von anorganischen oder organischen Protonensäuren durchgeführt. Als solche seien z.B. genannt: Salzsäure, Schwefelsäure, Trifluoressigsäure, Essigsäure, Ameisensäure.

Die Umsetzung erfolgt bei Temperaturen zwischen -20 und +50°C, vorzugsweise zwischen -10 und +20°C bei Normaldruck oder erhöhtem Druck. Es wird bevorzugt bei Normaldruck gearbeitet.

Die zur Durchführung des Verfahrens 3b als Ausgangsstoffe benötigten Dioxomorpholine sind bereits bekannt (z.B. WO 940 3441 A1; Tetrahedron Letters 1983, 24, 1921; Liebigs An. Chem.; 1982, 1952; Biological and Biomechanical Performance of Biomaterials, 1986, 245, Eds P. Christel, A. Maunier, A.J.C. Lee)

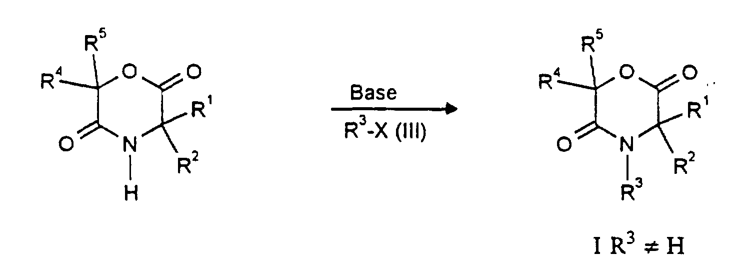

Die neuen Verbindungen der Formel (I) lassen sich nach den von N.L. Benoiton et al. für N-Methylaminosäuren angewendeten Verfahren (CAN. J. Chem., 1977, 55, 906; CAN. J. Chem., 1973, 51, 1915) herstellen.

Als Alkylierungsreagenzien kommen Alkylhalogenide in Frage, insbesondere Alkyliodide und -bromide.

Die Umsetzung erfolgt bei Temperaturen von 0 bis 150°C, bevorzugt bei 20 bis 100°C, besonders bevorzugt bei Raumtemperatur.

Als Verdünnungsmittel kommen alle inerten organischen Lösungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethylund Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile wie z.B. Acetonitril und Propionitril, Benzonitril, Glutarsäuredinitril, darüber hinaus Amide wie z.B. Dimethylformamid, Dimethylacetamid und N-Methylpyrrolidon, sowie Dimethylsulfoxid, Tetramethylensulfon und Hexamethylphosphorsäuretriamid.

Die Alkylierung wird in Gegenwart einer Base durchgeführt.

Als Basen kommen anorganische und organische Basen in Frage. Als Basen seien genannt: Alkali- und Erdalkalihydroxide, -carbonate, -hydrogencarbonate, -alkoholate, ferner Amine wie insbesondere tertiare Amine, z.B. Trimethylamin, Triethylamin, N-Methylmorpholin, Pyridin, Picoline, N-Ethylpyrrolidin, Diazabicyclo(4,3,0)-undecen (DBU), 1,4-Diazabicyclo(2,2,2)octan (DABCO), Diazabicyclo(3,2,0)nonen (DBN), Ethyl-diisopropylamin, NaH, organometallischen Basen wie "Butyllithium, Lithium Diisopropylamid (LDA), Lithium Tetramethylpiperidid (LTMP).

Die Verbindungen der Formeln (II) und die Basen werden werden im Verhältnis 1:1 bis 5:1 zueinander eingesetzt.

Nach erfolgter Umsetzung wird das Verdünnungsmittel abdestilliert und die Verbindungen der Formel (I) in üblicher Weise, z.B. chromatographisch, gereinigt.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Losungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Losungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfordernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl /Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsaureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Tragerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent

Zubereitungen, die vor Anwendung verdunnt werden, enthalten den Wirkstoff in Konzentrationen von 0,5 - 90 Gew -%, bevorzugt von 5 - 50 Gew-%

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 2 | 10 |
| 4 | 10 |
| 5 | 10 |
| 6 | 10 |

### Herstellungsbeispiele

### 1. Vorschrift zur Herstellung der Verbindungen der Formel (I) gemäß Verfahren 3a

Zu einer Lösung der Verbindung (II) (7,36 mmol) und Hünig-Base (25,6 mmol) in DMF (100 ml) wurde bei 0°C BOP-Cl (8,76 mmol) zugegeben und 24 Stunden bei Zimmertemperatur nachgerührt. Nach dieser Zeit wurden dieselben Mengen BOP-Cl und Base zugegeben und weitere 24 Stunden gerührt. DMF wurde im Vakuum abrotiert und der Rückstand mit Dichlormethan versetzt. Die Lösung wurde nacheinander mit 2 N Salzsäure, gesättigter Natriumhydrogencarbonatlösung und Wasser gewaschen, über Magnesiumsulfat getrocknet und eingeengt. Der Rückstand wurde säulenchromatographisch mit dem Laufmittel Cyclohexan-Ethylacetat 5:1 gereinigt. Es wurden Verbindungen der Formel (I) erhalten, in welcher die Substituenten die folgende Bedeutung haben:

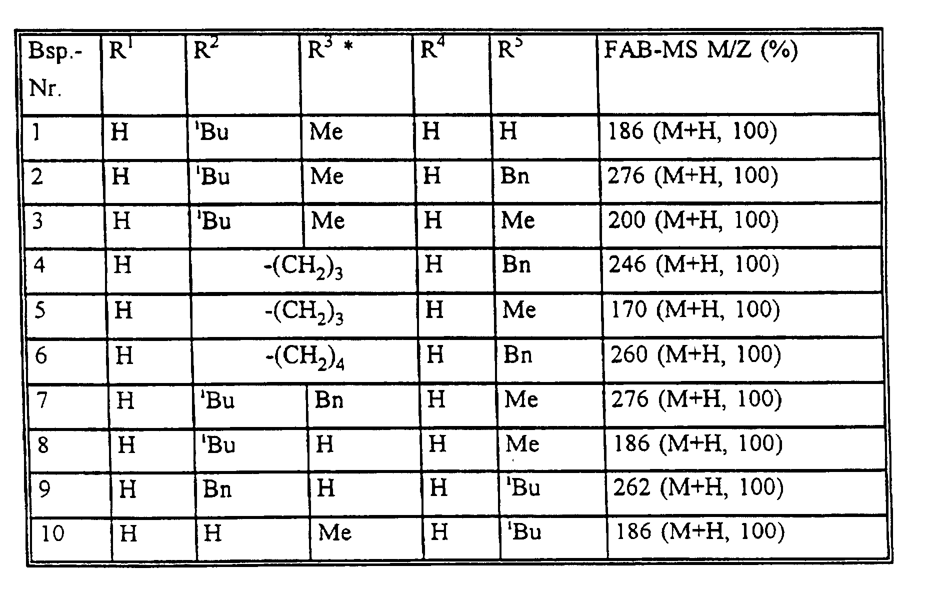

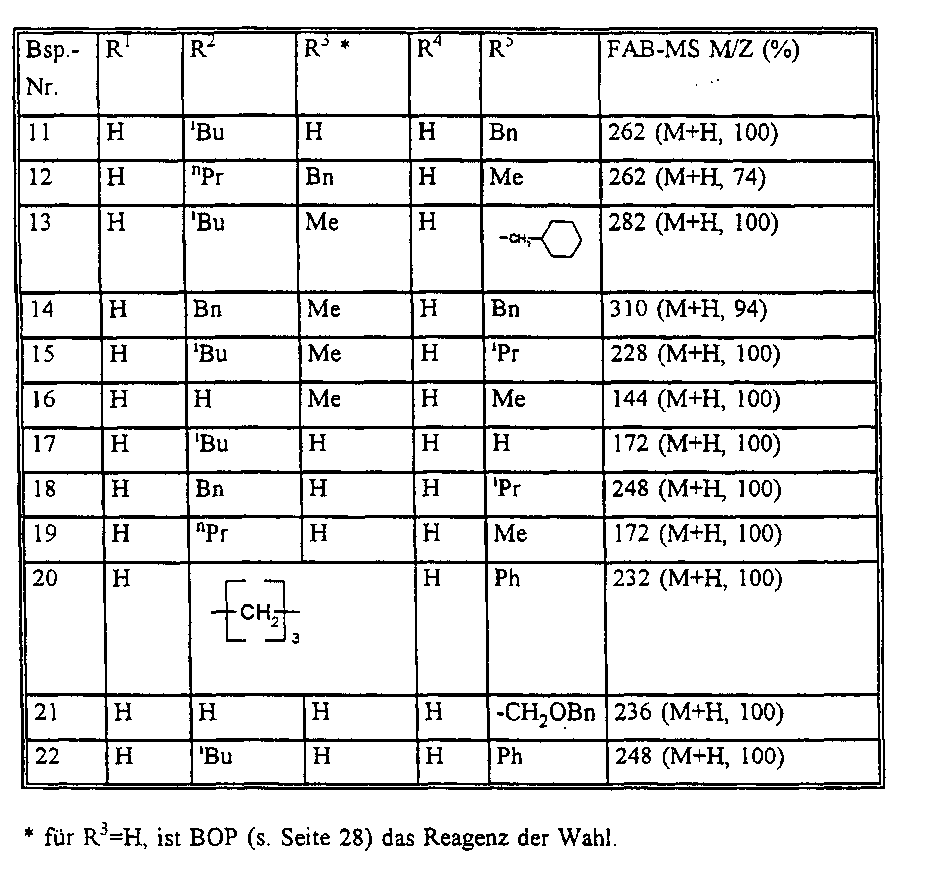

### 2. Allgemeine Vorschrift zur Herstellung der Verbindungen der Formel (I) gemäß Verfahren 3b

Zu einer Lösung von den Verbindungen (I) (R³=H, 10 mmol) und (III) (80 mmol) in THF (30 ml) wurde bei 0°C NaH (30 mmol) portionsweise zugegeben und 24 Stunden bei Zimmertemperatur nachgerührt. Man säuert die Lösung mit 5 % wäßriger Citronensäure an und extrahiert mit Methylenchlorid (2 x 100 ml). Die organische Phase wurde über Magnesiumsulfat getrocknet und im Vakuum eingeengt. Das Rohprodukt wurde säulenchromatographisch mit dem Laufmittel Cyclohexan-Ethylacetat gereinigt. Es wurden Verbindungen der Formel (I) erhalten, in denen die Substituenten die folgende Bedeutung haben:

| Bsp.-Nr. | R¹ | R² | R³ | R⁴ | R⁵ | physik Daten FAB-MS M/Z (%) |
|---|---|---|---|---|---|---|
| 3 | H | ¹Bu | Me | H | Me | 200 (M+H, 100) |
| 7 | H | ¹Bu | Bn | H | Me | 276 (M+H, 100) |
| 23 | H | ¹Bu | Et | H | Me | |
| 24 | H | ¹Bu | ⁿPr | H | Me | |
| 25 | H | Me | Me | H | Me | |
| 26 | H | Me | Et | H | Me | |
| 27 | H | ¹Bu | Bn | H | H | |
| 28 | H | ¹Bu | Et | H | H | |
| 29 | H | Me | Me | H | H | |
| 30 | H | Me | Et | H | H | |

### 3. Vorschrift zur Herstellung der Verbindungen der Formel (II) gemäß Verfahren 5a

In eine Lösung des tert.-Butylesters der Formel (IV) (1,61 mmol) in Dichlormethan (40 ml) wurde bei 0°C 1,5 h HCl-Gas eingeleitet. Anschließend wurde auf Raumtemperatur aufgewärmt und 12 h nachgerührt. Die Lösung wurde einrotiert und im Hochvakuum getrocknet. Der in Wasser gelöste Rückstand wurde in eine Suspension eines basischen Ionenaustauschers (0,60 g) in 5 ml Wasser eingetropft, 3 h gerührt, abfiltriert und eingeengt. Nach Trocknung im Hochvakuum wurde das Produkt ohne weitere Reinigung umgesetzt.

Gemäß dieser Vorschrift wurden Verbindungen der Formel (II) erhalten, in welcher die Substituenten die folgende Bedeutung haben:

| Bsp.-Nr. | R¹ | R² | R³ | R⁴ | R⁵ |
|---|---|---|---|---|---|
| II-1 | H | ¹Bu | Me | H | H |
| II-2 | H | ¹Bu | Me | H | Bn |
| II-3 | H | ¹Bu | Me | H | Me |
| II-4 | H | ¹Bu | Bn | H | Me |
| II-5 | H | H | Me | H | ¹Bu |

### 4. Vorschrift zur Herstellung der Verbindungen der Formel (II) gemäß Verfahren 5b

In eine Lösung des tert.-Butylesters der Formel (V) (1,61 mmol) in Dichlormethan (40 ml) wurde bei 0°C 1,5 h HCI-Gas eingeleitet. Anschließend wurde auf Raumtemperatur aufgewärmt und 12 h nachgerührt. Die Lösung wurde einrotiert und im Hochvakuum getrocknet. Der in Wasser gelöste Rückstand wurde in eine Suspension eines basischen Ionenaustauschers (0,60 g) in 5 ml Wasser eingetropft, 3 h gerührt, abfiltriert und eingeengt. Nach Trocknung im Hochvakuum wurde das Produkt ohne weitere Reinigung umgesetzt.

Gemäß dieser Vorschrift wurden Verbindungen der Formel (II) erhalten, in welcher die Substituenten die folgende Bedeutung haben:

| Bsp.-Nr. | R¹ | R² | R³ | R⁴ | R⁵ |
|---|---|---|---|---|---|
| II-6 | H | -(CH₂)₃ | | H | Bn |
| II-7 | H | -(CH₂)₃ | | H | Me |
| II-8 | H | -(CH₂)₄ | | H | Bn |
| II-9 | H | ¹Bu | H | H | ¹Bu |
| II-10 | H | Bn | H | H | ¹Bu |

### 5. Vorschrift zur Herstellung der Verbindungen der Formel (IV) gemäß Verfahren 7

Eine Lösung einer Verbindung der Formel (VI) (9,50 mmol) in Dioxan (50 ml) wurde in Gegenwart von Pd(OH)₂/C (20 %; 600 mg) bis zur Beendigung der Wasserstoffaufnahme hydriert (ca. 2 Stunden). Nach Abfiltrieren des Katalysators fiel Verbindung (VII) in nahezu quantitativer Ausbeute an, die ohne zusätzliche Reinigung weiter umgesetzt wurde.

Gemäß dieser Vorschrift wurden Verbindungen der Formel (IV) erhalten, in welcher die Substituenten die folgende Bedeutung haben:

| Bsp.-Nr. | R¹ | R² | R³ | R⁴ | R⁵ |
|---|---|---|---|---|---|
| IV-1 | H | ¹Bu | Me | H | H |
| IV-2 | H | ¹Bu | Me | H | Bn |
| IV-3 | H | ¹Bu | Me | H | Me |
| IV-4 | H | ¹Bu | Bn | H | Me |
| IV-5 | H | H | Me | H | ¹Bu |

### 6. Verfahren zur Herstellung der Verbindungen der Formel (V) gemäß Verfahren 9

Die Aminosäure der Formel (VII) (0,40 mol) wurde in 1400 ml Ethanol und 800 ml Wasser gelöst, mit einer 20 %igen Caesiumcarbonatlösung (390 ml) versetzt und 2 Stunden bei Raumtemperatur gerührt. Anschließend wurde eingeengt, in Wasser (2000 ml) gelöst und gefriergetrocknet. 0,40 mol dieses Caesiumsalzes wurden in 1000 ml Dimethylformamid vorgelegt, bei Raumtemperatur mit 0,40 mol der Chlorcarbonsäure der Formel (VIII) versetzt und 20 Stunden bei Raumtemperatur gerührt. Die Lösung wurde eingeengt, der Rückstand in Wasser (1000 ml) eingegossen, viermal mit Ethylacetat extrahiert, über Natriumsulfat getrocknet und eingeengt. Der Rückstand wurde ohne zusätzliche Reinigung weiter umgesetzt.

Analog wurden die Verbindungen der Formel (V) erhalten, in welcher die Substituenten die folgende Bedeutung haben:

| Bsp.-Nr. | R¹ | R² | R³ | R⁴ | R⁵ |
|---|---|---|---|---|---|
| V-1 | H | -(CH₂)₃ | | H | Bn |
| V-2 | H | -(CH₂)₃ | | H | Me |
| V-3 | H | -(CH₂)₄ | | H | Bn |
| V-4 | H | ¹Bu | H | H | ¹Bu |
| V-5 | H | Bn | H | H | ¹Bu |

### 7. Vorschrift zur Herstellung der Verbindungen der Formel (VI) gemäß Verfahren 11

Die Aminosäure der Formel (IX) (0,40 mol) wurde in 1400 ml Ethanol und 800 ml Wasser gelöst, mit einer 20 %igen Caesiumcarbonatlösung (390 ml) versetzt und 2 Stunden bei Raumtemperatur gerührt. Anschließend wurde eingeengt, in Wasser (2000 ml) gelöst und gefriergetrocknet. 0,40 mol dieses Caesiumsalzes wurden in 1000 ml Dimethylformamid vorgelegt, bei Raumtemperatur mit 0,40 mol der Chlorcarbonsäure der Formel (VIII) versetzt und 20 Stunden bei Raumtemperatur gerührt. Die Lösung wurde eingeengt, der Rückstand in Wasser (1000 ml) eingegossen, viermal mit Ethylacetat extrahiert, über Natriumsulfat getrocknet und eingeengt. Der Rückstand wurde ohne zusätzliche Reinigung weiter umgesetzt.

Analog wurden die Verbindungen der Formel (VI) erhalten, in welcher die Substituenten die folgende Bedeutung haben:

| Bsp.-Nr. | R¹ | R² | R³ | R⁴ | R⁵ |
|---|---|---|---|---|---|
| VI-1 | H | ¹Bu | Me | H | H |
| VI-2 | H | ¹Bu | Me | H | Bn |
| VI-3 | H | ¹Bu | Me | H | Me |
| VI-4 | H | ¹Bu | Bn | H | Me |
| VI-5 | H | H | Me | H | ¹Bu |

### Beispiel zur Durchführung der Nitrierung

### 3-Isobutyl-4-methyl-6-(2,4-dinitrophenyl)-methyl-morpholin-2,5-dion

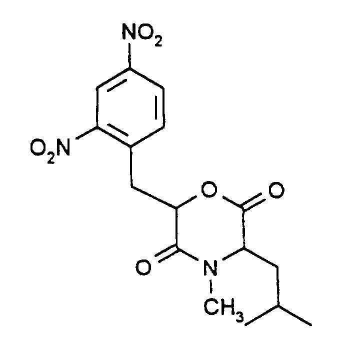

Zu einer Lösung von Eis gekühlter Salpetersäure (20 ml, 98%ig) wurde 3-Isobutyl-4-methyl-6-benzyl-morpholin-2,5-dion (2,00 g, 7 mmol) zugegeben und 0,5 Stunden bei 0°C, anschließend 1 Stunde bei Zimmertemperatur nachgerührt. Die Lösung wurde auf Eis gegossen und dann mit Dichlormethan extrahiert. Die organische Phase wurde über Magnesiumsulfat getrocknet und im Vakuum eingeengt. Das Rohprodukt wurde säulenchromatographisch mit dem Laufmittel Cyclohexan-Ethylacetat (5:1) gereinigt. Ausbeute = 1,26 g, 56 % der Theorie.

Es wurden Verbindungen der Formel (I) erhalten, in welcher die Substituenten die folgende Bedeutung haben

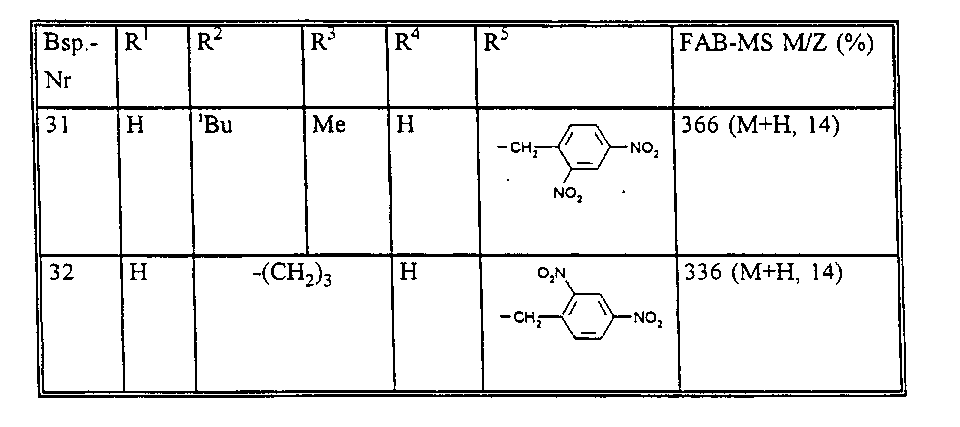

### Beispiel zur Reduktion der Nitrogruppe am Phenylring

### Beispiel 33

### 3-Isobutyl-4-methyl-6-(2-amino-4-nitrophenyl)methyl-morpholin-2,5-dion und 3-Isobutyl-4-methyl-6-(4-amino-2-nitrophenyl)methyl-morpholin-2,5-dion

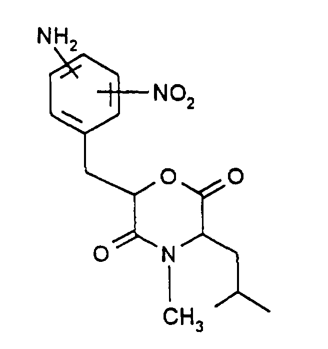

3-Isobutyll-4-methyl-6-(2,4-dinitrophenyl)-methyl-morpholin-2,5-dion (0,50 g, 1,37 mmol) wurde in Dioxan (40 ml) in Gegenwart von Pd(OH)₂ (20 mol-%) hydriert bis Ausgangsmaterial konnte mittel Dünnschichtchromatographie nicht mehr detektiert werden. Der Katalysator wurde abfiltriert und das Lösungsmittel im Vakuum eingeengt. Die Rohprodukte wurden säulenchromatographisch gereinigt.

Gesamtausbeute = 0,20g, 49 % der Theorie, FAB-MS M/Z (%) 336 (M+H, 14)

### Beispiel 34

### 3-Isobutyl-4-methyl-6-(2,4-diaminophenyl)methyl-morpholin-2,5-dion

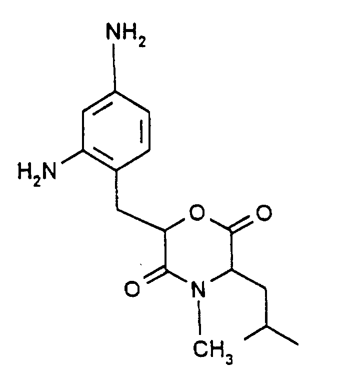

3-Isobutyl-4-methyl-6-(2,4-dinotrophenyl)methyl-morpholin-2,5-dion (1,20 g, 3,28 mmol) wurde in Methanol (116 ml) und Wasser (1,16 ml in Gegenwart von Ranay-Nickel (3,8 g) bis zur Beendigung der Wasserstoffaufnahme hydriert. Der Katalysator wurde abfiltriert und das Lösungsmittel im Vakuum abrotiert.

Ausbeute = 0,84 g, 84 % der Theorie, FAB-MS M/Z (%) 306 (M+H, 40)

### Beispiel 35

### 3-Isobutyl-4-methyl-6-(2,4-diacetamidophenyl)methyl-morpholin-2,5-dione

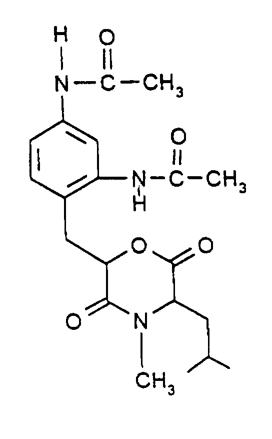

3-Isobutyl-4-methyl-6-(2,4-diaminophenyl)-methyl-morpholine-2,5-dione (34) (0,50 g, 1,7 mmol) und Triethylamin (0,50 ml, 3,6 mmol) wurden in Dichlormethan (20 ml) vorgelegt. Acetylchlorid (0,26 ml, 3,6 mmol) wurde bei 20°C zugetropft und die Lösung auf Rückfluß erhitzt für 24 Stunden. Die Lösung wurde dann mit Dichlormethan (50 ml) verdünnt und nacheinander mit Salzsäure (2 ml, 10 ml) und gesättigter Natriumhydrogencarbonatlösung gewaschen. Die organische Phase wurde über Natriumsulfat getrocknet und in Vakuum absorbiert. Das Rohprodukt wurde säulenchromatographisch gereinigt.
(Laufmittel Essigester: Cyclohexan 1:10)
Ausbeute = 0,40 g, 63 % der Theorie, FAB.MS M/Z (%) 390 (M+H, 38)

### Beispiel 36

### 3-Isobutyl-4-methyl-6-[2,4-bis-(4-Chlorbenzamido)phenyl]methyl-morpholin-2,5-dion

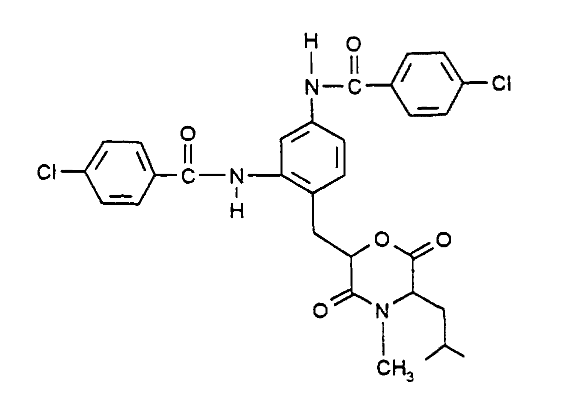

Beispiel 36 wurde aus Verbindung 34 (0,50 g) und 4-Chlorbenzoylchlorid (0,46 ml) analog zu Beispiel 35 hergestellt.

Ausbeute = 0,45 g, 45 % der Theorie, FAB-MS M/Z (%) 582 (M+H, 10)

## Patentansprüche

1. Verwendung von Dioxomorpholinen der Formel (I) 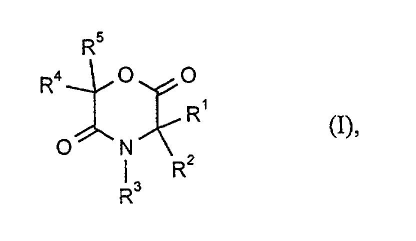 in welcher
R¹ und R² unabhängig voneinander für Wasserstoff, geradkettiges oder verzweigtes Alkyl, Alkenyl, Cycloalkyl, Cycloalkylalkyl sowie für gegebenenfalls substituiertes Aryl, Arylalkyl, Heteroaryl, Heteroarylalkyl stehen, die gegebenenfalls substituiert sind,
R¹ und R² gemeinsam für einen spirocyclischen Rest stehen, der gegebenenfalls substituiert ist,
R³ für Wasserstoff, geradkettiges oder verzweigtes Alkyl, Cycloalkyl, Aralkyl, Aryl, Heteroaryl, Heteroarylalkyl, die gegebenenfalls substituiert sind, steht,
R² und R³ gemeinsam mit den Atomen, an die sie gebunden sind, für einen 5- oder 6-gliedrigen Ring stehen, der gegebenenfalls substituiert sein kann,
R⁴ und R⁵ unabhängig voneinander für Wasserstoff, geradkettiges oder verzweigtes Alkyl, Alkenyl, Cycloalkyl, Cycloalkylalkyl sowie für gegebenenfalls substituiertes Aryl, Arylalkyl, Heteroaryl, Heteroarylalkyl stehen, die gegebenenfalls substituiert sind,
R⁴ und R⁵ gemeinsam für einen spirocyclischen Rest stehen, der gegebenenfalls substituiert ist
sowie deren optische Isomere und Racemate,
zur Herstellung von Mitteln zur Bekämpfung von Endoparasiten in der Medizin und Tiermedizin, wobei die gegebenenfalls substituierten Reste einen oder mehrere gleiche oder verschiedene Substituenten tragen, die ausgewählt sind aus der Gruppe: Alkyl mit 1 bis 4 Kohlenstoffatomen, Alkoxy mit 1 bis 4 Kohlenstoffatomen; Alkylthio mit 1 bis 4 Kohlenstoffatomen; Halogenalkyl mit 1 bis 4 Kohlenstoffatomen und 1 bis 5 gleichen oder verschiedenen Halogenatomen; Hydroxy; Halogen; Cyano; Nitro; Amino; Monoalkyl- und Dialkylamino mit 1 bis 4 Kohlenstoffatomen je Alkylgruppe; Alkylcarbonylamino mit 1 bis 4 Kohlenstoffatomen im Alkylteil; Benzoylamino, wobei Alkylcarbonylamino und Benzoylamino jeweils im Acylteil mit Halogen, Nitro, Trifluormethyl, Trifluormethoxy oder Trifluormethylthio substituiert sein können; Carboxyl; Carbalkoxy mit 2 bis 4 Kohlenstoffatomen, Alkylsulfinyl mit 1 bis 4 Kohlenstoffatomen, Halogenalkylsulfinyl mit 1 bis 4 Kohlenstoffatomen und 1 bis 5 Halogenatomen; Sulfonyl; Alkylsulfonyl mit 1 bis 4 Kohlenstoffatomen; Halogenalkylsulfonyl mit 1 bis 4 Kohlenstoffatomen und 1 bis 5 Halogenatomen; Arylsulfonyl mit 6 bis 10 Arylkohlenstoffatomen, Acyl, Aryl, Aryloxy, Heteroaryl, Heteroaryloxy, die ihrerseits einen der oben genannten Substituenten tragen können, sowie der Formiminorest -(CH)=N-O-Alkyl;
und wobei Alkyl allein oder als Bestandteil eines Restes geradkettiges oder verzweigtes Alkyl mit 1 bis 9 Kohlenstoffatomen bedeutet; Alkenyl allein oder als Bestandteil eines Restes geradkettiges oder verzweigtes Alkenyl mit 2 bis 20 Kohlenstoffatomen bedeutet; Cycloalkyl mono-, di- und tricyclisches Cycloalkyl mit 3 bis 10 Kohlenstoffatomen bedeutet; Aryl Phenyl oder Naphthyl bedeutet; Arylalkyl 6 bis 10 Kohlenstoffatome im Arylteil und 1 bis 4 Kohlenstoffatome im Alkylteil, der geradkettig oder verzweigt sein kann, aufweist; Heteroaryl für einen 5- bis 7-gliedrigen Ring mit 1 bis 3 gleichen oder verschiedenen Heteroatomen ausgewählt aus Sauerstoff, Schwefel und Stickstoff steht.

2. Verwendung von Dioxomorpholinen der Formel (I) gemäß Anspruch 1,
in welcher
R¹ und R² unabhängig voneinander für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl stehen,
R¹ und R² gemeinsam für einen spirocyclischen Rest stehen,
R³ für Wasserstoff, geradkettiges oder verzweigtes Alkyl, Cycloalkyl, Aralkyl, Aryl, Heteroaryl, Heteroarylalkyl, die gegebenenfalls substituiert sind, steht,
R² und R³ gemeinsam mit den Atomen, an die sie gebunden sind, für einen 5- oder 6-gliedrigen Ring stehen, der gegebenenfalls substituiert sein kann,
R⁴ und R⁵ unabhängig voneinander für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl stehen,
R⁴ und R⁵ gemeinsam für einen spirocyclischen Rest stehen,
sowie deren optische Isomere und Racemate.

3. Dioxomorpholine der Formel (I) 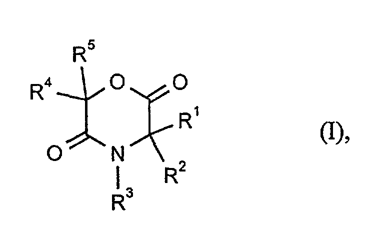 in welcher
R¹ für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl steht,
R² für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfmylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl steht,
R¹ und R² können zusammen für einen spirocyclischen Rest stehen,
R³ für geradkettiges oder verzweigtes Alkyl, Cycloalkyl, Aralkyl, Aryl, Heteroaryl, Heteroarylalkyl, die gegebenenfalls substituiert sind, steht,
R⁴ für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl steht,
R⁵ für Wasserstoff, geradkettiges oder verzweigtes Alkyl mit bis zu 8 Kohlenstoffatomen, Halogenalkyl, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie für gegebenenfalls substituiertes Aryl, Arylalkyl, Heteroaryl, Heteroarylalkyl steht,
R⁴ und R⁵ stehen gemeinsam für einen spirocyclischen Rest,
mit Ausnahme solcher Verbindungen, in denen gleichzeitig
a)
R¹ Wasserstoff bedeutet,
R² Wasserstoff, Methyl, Benzyl oder Isopropyl bedeutet und
R³ Methyl oder alkylsubstituiertes Phenyl bedeutet,
oder
b)
R¹ Wasserstoff bedeutet,
R² sec.-Butyl oder Isobutyl bedeutet,
R³ Methyl bedeutet,
R⁴ Wasserstoff bedeutet und
R⁵ Isopropyl bedeutet,
oder
c)
R¹ Wasserstoff bedeutet,
R² Isopropyl bedeutet,
R³ Wasserstoff bedeutet,
R⁴ Wasserstoff bedeutet und
R⁵ Isopropyl bedeutet,
wobei die gegebenenfalls substituierten Reste einen oder mehrere gleiche oder verschiedene Substituenten tragen, die ausgewählt sind aus der Gruppe: Alkyl mit 1 bis 4 Kohlenstoffatomen, Alkoxy mit 1 bis 4 Kohlenstoffatomen; Alkylthio mit 1 bis 4 Kohlenstoffatomen; Halogenalkyl mit 1 bis 4 Kohlenstoffatomen und 1 bis 5 gleichen oder verschiedenen Halogenatomen; Hydroxy; Halogen; Cyano; Nitro; Amino; Monoalkyl- und Dialkylamino mit 1 bis 4 Kohlenstoffatomen je Alkylgruppe; Alkylcarbonylamino mit 1 bis 4 Kohlenstoffatomen im Alkylteil; Benzoylamino, wobei Alkylcarbonylamino und Benzoylamino jeweils im Acylteil mit Halogen, Nitro, Trifluormethyl, Trifluormethoxy oder Trifluormethylthio substituiert sein können; Carboxyl; Carbalkoxy mit 2 bis 4 Kohlenstoffatomen, Alkylsulfinyl mit 1 bis 4 Kohlenstoffatomen, Halogenalkylsulfinyl mit 1 bis 4 Kohlenstoffatomen und 1 bis 5 Halogenatomen; Sulfonyl; Alkylsulfonyl mit 1 bis 4 Kohlenstoffatomen; Halogenalkylsulfonyl mit 1 bis 4 Kohlenstoffatomen und 1 bis 5 Halogenatomen; Arylsulfonyl mit 6 bis 10 Arylkohlenstoffatomen, Acyl, Aryl, Aryloxy, Heteroaryl, Heteroaryloxy, die ihrerseits einen der oben genannten Substituenten tragen können, sowie der Formiminorest -(CH)=N-O-Alkyl;
und wobei Alkyl allein oder als Bestandteil eines Restes geradkettiges oder verzweigtes Alkyl mit 1 bis 9 Kohlenstoffatomen bedeutet; Alkenyl allein oder als Bestandteil eines Restes geradkettiges oder verzweigtes Alkenyl mit 2 bis 20 Kohlenstoffatomen bedeutet; Cycloalkyl mono-, di- und tricyclisches Cycloalkyl mit 3 bis 10 Kohlenstoffatomen bedeutet; Aryl Phenyl oder Naphthyl bedeutet; Arylalkyl 6 bis 10 Kohlenstoffatome im Arylteil und 1 bis 4 Kohlenstoffatome im Alkylteil, der geradkettig oder verzweigt sein kann, aufweist; Heteroaryl für einen 5- bis 7-gliedrigen Ring mit 1 bis 3 gleichen oder verschiedenen Heteroatomen ausgewählt aus Sauerstoff, Schwefel und Stickstoff steht;
sowie deren optische Isomere und Racemate.

4. Verfahren zur Herstellung der Dioxomorpholine der Formel (I) gemäß Anspruch 3 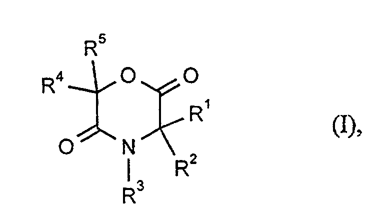 in welcher
die Reste R¹ bis R⁵ die unter Anspruch 3 angegebene Bedeutung haben,
**dadurch gekennzeichnet, daß** man
a) offenkettige Depsipeptide der Formel (II) 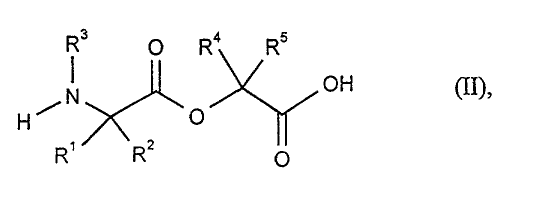 in welcher
die Reste R¹ bis R⁵ die oben angegebene Bedeutung haben,
in Gegenwart eines Verdünnungsmittels und in Gegenwart eines Kopplungsreagenzes cyclisiert,
oder
b) Verbindungen der Formel (I) 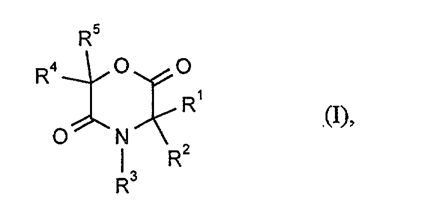 in welcher
R³ für Wasserstoff steht, und
R¹, R², R⁴ und R⁵ die oben angegebene Bedeutung haben
mit Verbindungen der Formel (III)
X-R³ (III),
in welcher
R³ für geradkettiges oder verzweigtes Alkyl, Cycloalkyl, Aralkyl oder Heteroarylalkyl, die gegebenenfalls substituiert sein können, steht,
X für I, Cl oder Br steht,
in Gegenwart eines Verdünnungsmittels und einer Base umsetzt.

5. Offenkettige Depsipeptide der Formel (II) 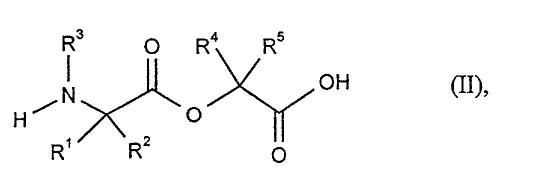 in welcher
R¹ bis R⁵ in Anspruch 3 angegebene Bedeutungen haben.

6. Verfahren zur Herstellung der offenkettigen Depsipeptide der Formel (II) 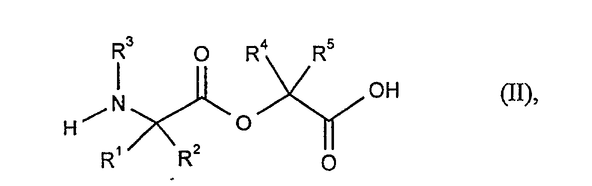 in welcher
R¹ bis R⁵ die in Anspruch 3 angegebene Bedeutung besitzen,
**dadurch gekennzeichnet, daß** man
a) Verbindungen der Formel (IV) 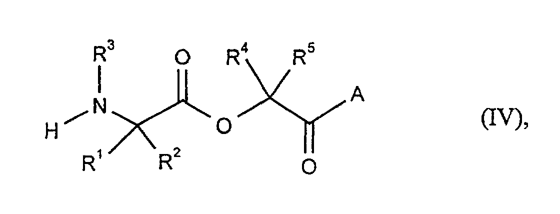 in welcher
A für tert.-Butoxy steht,
R¹ bis R⁵ die oben angegebenen Bedeutungen besitzen,
in Gegenwart eines Verdünnungsmittels und einer Protonensäure verseift,
oder
b) Verbindungen der Formel (V) 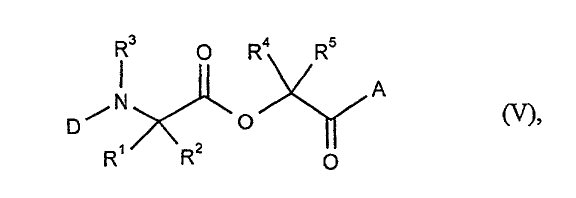 in welcher
A für tert.-Butoxy steht,
D für tert.-Butoxycarbonyl (-CO₂^{t}Bu) steht,
R¹ bis R⁵ die oben angegebenen Bedeutungen haben,
in Gegenwart eines Verdünnungsmittels und einer Protonensäure verseift.

7. Verbindungen der Formel (IV) 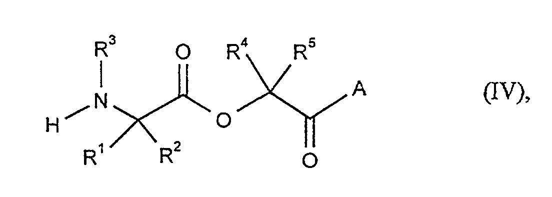 in welcher
A für tert.-Butoxy steht,
R¹ bis R⁵ die in Anspruch 3 angegebenen Bedeutungen haben,
mit der Maßgabe, daß wenn
R¹ und R⁴ für Wasserstoff stehen
R² nicht Wasserstoff, geradkettiges oder verzweigtes Alkyl, Alkenyl, Cycloalkyl, Aryl, Arylalkyl oder Heteroarylalkyl, die jeweils gegebenenfalls substituiert sind, bedeutet.

8. Verfahren zur Herstellung von Verbindungen der Formel (IV) 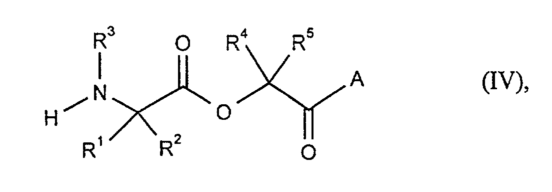 in welcher
A für tert.-Butoxy steht,
R¹ bis R⁵ die in Anspruch 7 angegebenen Bedeutungen haben,
**dadurch gekennzeichnet, daß** man Verbindungen der Formel (VI) 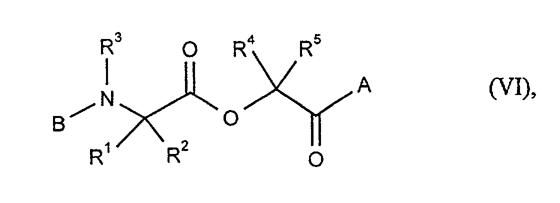 in welcher
A für tert.-Butoxy,
B für Benzyl,
R¹ bis R⁵ die oben angegebene Bedeutung haben,
in Gegenwart eines Verdünnungsmittels und eines Katalysators hydrogenolisiert.

9. Verbindungen der Formel (V) 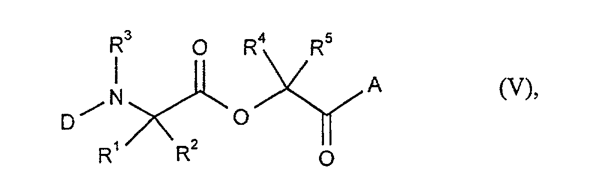 in welcher
A für tert.-Butoxy steht,
D für tert.-Butoxycarbonyl steht,
R¹ bis R⁵ die in Anspruch 3 angegebenen Bedeutungen haben.

10. Verfahren zur Herstellung von Verbindungen der Formel (V) 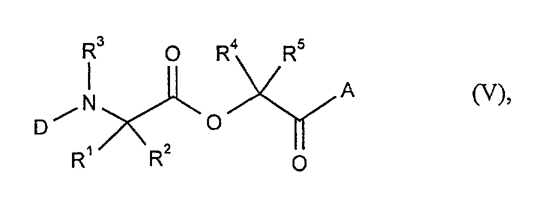 in welcher
A für tert.-Butoxy steht,
D für tert.-Butoxycarbonyl steht,
R¹ bis R⁵ die in Anspruch 3 angegebenen Bedeutungen besitzen,
**dadurch gekennzeichnet, daß** man Verbindungen der Formel (VII) 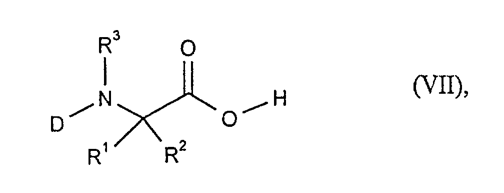 in welcher
D für tert.-Butyloxycarbonyl steht,
R¹ bis R³ die oben angegebene Bedeutung besitzen, in Form ihres Alkalimetallsalzes, bevorzugt ihres Caesiumsalzes und eine α-Halogencarbonsäure der Formel (VIII) 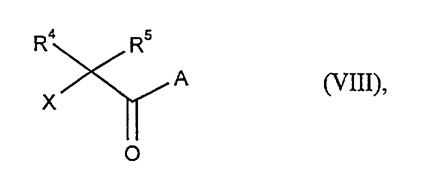 in welcher
A für tert.-Butoxy steht,
X für Cl oder Br und
R⁴ und R⁵ die oben angegebene Bedeutung haben,
in Gegenwart eines Verdünnungsmittels umsetzt.

11. Verbindungen der Formel (VI) 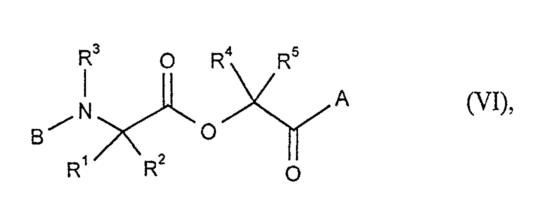 in welcher
A für tert.-Butoxy,
B für Benzyl steht,
R¹ bis R⁵ die in Anspruch 3 angegebene Bedeutung besitzen,
mit der Maßgabe, daß wenn
R¹ und R⁴ für Wasserstoff stehen
R² nicht Wasserstoff, geradkettiges oder verzweigtes Alkyl, Alkenyl, Cycloalkyl, Aryl, Arylalkyl oder Heteroarylalkyl, die jeweils gegebenenfalls substituiert sind, bedeutet.

12. Verfahren zur Herstellung von Verbindungen der Formel (VI) 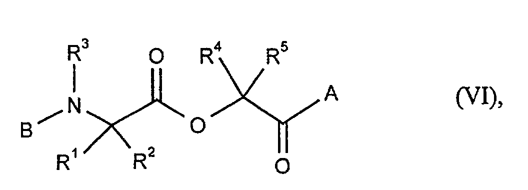 in welcher
A für tert.-Butoxy steht,
B für Benzyl sowie
R¹ bis R⁵ die in Anspruch 11 angegebene Bedeutung haben,
**dadurch gekennzeichnet, daß** man Verbindungen der Formel (IX) 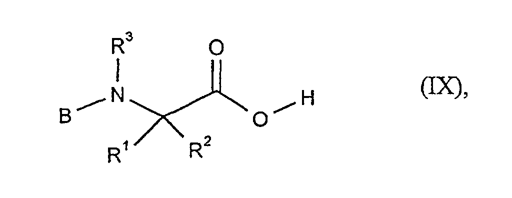 in welcher
B für Benzyl steht,
R¹ bis R³ die oben angegebene Bedeutung haben,
in Form ihres Alkalimetallsalzes, bevorzugt ihres Caesiumsalzes, mit einem α-Halogencarbonsäurederivat der Formel (VIII) 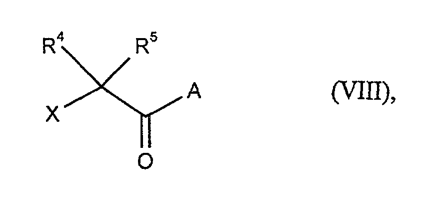 in welcher
X für Cl, Br steht,
A für tert.-Butoxy,
R⁴ und R⁵ die oben angegebene Bedeutung besitzen,
umsetzt.

13. Endoparasitizide Mittel, **gekennzeichnet durch** einen Gehalt an mindestens einem Dioxomorpholin der Formel (I) gemäß Anspruch 3.

14. Verfahren zur Herstellung von endoparasitiziden Mitteln, **dadurch gekennzeichnet, daß** man Dioxomorpholine der Formel (I) gemäß Anspruch 3 mit Streckmitteln und/oder oberflächenaktiven Mitteln vermischt.

## Claims

1. Use of dioxomorpholines of the formula (I) 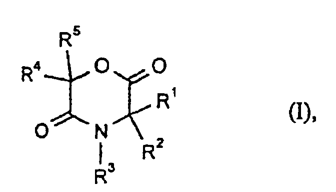 in which
R¹ and R² independently of one another represent hydrogen, straight-chain or branched alkyl, alkenyl, cycloalkyl, cycloalkylalkyl, as well as optionally substituted aryl, aralkyl, heteroaryl or heteroarylalkyl which are optionally substituted,
R¹ and R² together represent a spirocyclic radical which is optionally substituted,
R³ represents hydrogen, straight-chain or branched alkyl, cycloalkyl, aralkyl, aryl, heteroaryl or heteroarylalkyl, which are optionally substituted,
R² and R³ together with the atoms to which they are bonded represent a 5- or 6-membered ring, which can optionally be substituted,
R⁴ and R⁵ independently of one another represent hydrogen, straight-chain or branched alkyl, alkenyl, cycloalkyl, cycloalkylalkyl, as well as optionally substituted aryl, arylalkyl, heteroaryl or heteroarylalkyl, which are optionally substituted
R⁴ and R⁵ together represent a spirocyclic radical, which is optionally substituted
and optical isomers and racemates thereof,
for preparing compositions for combating endoparasites in human and veterinary medicine, the optionally substituted radicals carrying one or more identical or different substituents selected from the following group: alkyl having 1 to 4 carbon atoms, alkoxy having 1 to 4 carbon atoms; alkylthio having 1 to 4 carbon atoms; halogenoalkyl having 1 to 4 carbon atoms and 1 to 5 identical or different halogen atoms; hydroxyl; halogen; cyano; nitro; amino; monoalkyl- and dialkylamino having 1 to 4 carbon atoms per alkyl group; alkylcarbonylamino having 1 to 4 carbon atoms in the alkyl moiety; benzoylamino, it being possible for alkylcarbonylamino and benzylamino each to be substituted in the acyl moiety by halogen, nitro, trifluoromethyl, trifluoromethoxy or trifluoromethylthio; carboxyl; carbalkoxy having 2 to 4 carbon atoms, alkylsulphinyl having 1 to 4 carbon atoms, halogenoalkylsulphinyl having 1 to 4 carbon atoms and 1 to 5 halogen atoms; sulphonyl; alkylsulphonyl having 1 to 4 carbon atoms; halogenoalkylsulphonyl having 1 to 4 carbon atoms and 1 to 5 halogen atoms; arylsulphonyl having 6 to 10 aryl carbon atoms, acyl, aryl, aryloxy, heteroaryl, heteroaryloxy, which may in turn carry one of the abovementioned substituents, and also the formimino radical -(CH)=N-O-alkyl;
and alkyl, alone or as part of a radical, denoting straight-chain or branched alkyl having 1 to 9 carbon atoms; alkenyl, alone or as part of a radical, denoting straight-chain or branched alkenyl having 2 to 20 carbon atoms; cycloalkyl denoting monocyclic, dicyclic and tricyclic cycloalkyl having 3 to 10 carbon atoms; aryl denoting phenyl or naphthyl; arylalkyl having 6 to 10 carbon atoms in the aryl moiety and 1 to 4 carbon atoms in the alkyl moiety, which may be straight-chain or branched; heteroaryl representing a 5- to 7-membered ring having 1 to 3 identical or different heteroatoms selected from oxygen, sulphur and nitrogen.

2. Use of dioxomorpholines of the formula (I) according to Claim 1,
in which
R¹ and R² independently of one another represent hydrogen, straight-chain or branched alkyl having up to 8 carbon atoms, halogenoalkyl, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, guanidinoalkyl, which can optionally be substituted by one or two benzyloxycarbonyl radicals or by one, two, three or four alkyl radicals, alkoxycarbonylaminoalkyl, 9-fluorenylmethoxycarbonyl(Fmoc)-aminoalkyl, alkenyl, cycloalkyl or cycloalkylalkyl, or represent optionally substituted aryl, arylalkyl, heteroaryl or heteroarylalkyl,
R¹ and R² together represent a spirocyclic radical,
R³ represents hydrogen, straight-chain or branched alkyl, cycloalkyl, aralkyl, aryl, heteroaryl or heteroarylalkyl, which are optionally substituted,
R² and R³ together with the atoms to which they are bonded represent a 5- or 6-membered ring, which can optionally be substituted,
R⁴ and R⁵ independently of one another represent hydrogen, straight-chain or branched alkyl having up to 8 carbon atoms, halogenoalkyl, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, guanidinoalkyl, which can optionally be substituted by one or two benzyloxycarbonyl radicals or by one, two, three or four alkyl radicals, alkoxycarbonylaminoalkyl, 9-fluorenylmethoxycarbonyl(Fmoc)-aminoalkyl, alkenyl, cycloalkyl or cycloalkylalkyl, or represent optionally substituted aryl, arylalkyl, heteroaryl or heteroarylalkyl,
R⁴ and R⁵ together represent a spirocyclic radical,
and optical isomers and racemates thereof.

3. Dioxomorpholines of the formula (I) 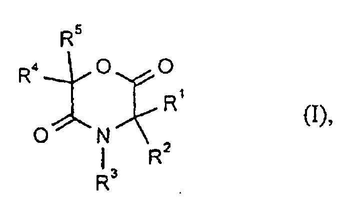 in which
R¹ represents hydrogen, straight-chain or branched alkyl having up to 8 carbon atoms, halogenoalkyl, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, guanidinoalkyl, which can optionally be substituted by one or two benzyloxycarbonyl radicals or by one, two, three or four alkyl radicals, alkoxycarbonylaminoalkyl, 9-fluorenylmethoxycarbonyl(Fmoc)aminoalkyl, alkenyl, cycloalkyl or cycloalkylalkyl, or represents optionally substituted aryl, arylalkyl, heteroaryl or heteroarylalkyl,
R² represents hydrogen, straight-chain or branched alkyl having up to 8 carbon atoms, halogenoalkyl, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, carboxyalkyl, alkoxy-carbonylalkyl, arylalkox ycarbonylalkyl, carbamoylalkyl, amino-alkyl, alkylaminoalkyl, dialkylaminoalkyl, guanidinoalkyl, which can optionally be substituted by one or two benzyloxycarbonyl radicals or by one, two, three or four alkyl radicals, alkoxycarbonylaminoalkyl, 9-fluorenylmethoxycarbonyl(Fmoc)aminoalkyl, alkenyl, cycloalkyl or cycloalkylalkyl, or represents optionally substituted aryl, arylalkyl, heteroaryl or heteroarylalkyl,
R¹ and R² together can represent a spirocyclic radical,
R³ represents straight-chain or branched alkyl, cycloalkyl, aralkyl, aryl, heteroaryl or heteroarylalkyl, which are optionally substituted,
R⁴ represents hydrogen, straight-chain or branched alkyl having up to 8 carbon atoms, halogenoalkyl, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, alkoxycarbonylalkyl, arylalkoxycarbonylalkyl, carbamoylalkyl, aminoalkyl, alkylaminoalkyl, dialkylaminoalkyl, guanidinoalkyl, which can optionally be substituted by one or two benzyloxycarbonyl radicals or by one, two, three or four alkyl radicals, alkoxycarbonylaminoalkyl, 9-fluorenylmethoxycarbonyl(Fmoc)aminoalkyl, alkenyl, cycloalkyl or cycloalkylalkyl, or represents optionally substituted aryl, arylalkyl, heteroaryl or heteroarylalkyl,
R⁵ represents hydrogen, straight-chain or branched alkyl having up to 8 carbon atoms, halogenoalkyl, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, alkoxycarbonylalkyl, arylalkoxycarbonylalkyl, carbamoylalkyl, aminoalkyl, alkylaminoalkyl, dialkylaminoalkyl, guanidinoalkyl, which can optionally be substituted by one or two benzyloxycarbonyl radicals or by one, two, three or four alkyl radicals, alkoxycarbonylaminoalkyl, 9-fluorenylmethoxycarbonyl(Fmoc)aminoalkyl, alkenyl, cycloalkyl or cycloalkylalkyl, or represents optionally substituted aryl, arylalkyl, heteroaryl or heteroarylalkyl,
R⁴ and R⁵ together represent a spirocyclic radical,
with the exception of those compounds in which simultaneously
a)
R¹ denotes hydrogen,
R² denotes hydrogen, methyl, benzyl or isopropyl and
R³ denotes methyl or alkyl-substituted phenyl,
or
b)
R¹ denotes hydrogen,
R² denotes sec-butyl or isobutyl,
R³ denotes methyl,
R⁴ denotes hydrogen and
R⁵ denotes isopropyl,
or
c)
R¹ denotes hydrogen,
R² denotes isopropyl,
R³ denotes hydrogen,
R⁴ denotes hydrogen and
R⁵ denotes isopropyl,
the optionally substituted radicals carrying one or more identical or different substituents selected from the following group: alkyl having 1 to 4 carbon atoms, alkoxy having 1 to 4 carbon atoms; alkylthio having 1 to 4 carbon atoms; halogenoalkyl having 1 to 4 carbon atoms and 1 to 5 identical or different halogen atoms; hydroxyl; halogen; cyano; nitro; amino; monoalkyl- and dialkylamino having 1 to 4 carbon atoms per alkyl group; alkylcarbonylamino having 1 to 4 carbon atoms in the alkyl moiety; benzoylamino, it being possible for alkylcarbonylamino and benzylamino each to be substituted in the acyl moiety by halogen, nitro, trifluoromethyl, trifluoromethoxy or trifluoromethylthio; carboxyl; carbalkoxy having 2 to 4 carbon atoms, alkylsulphinyl having 1 to 4 carbon atoms, halogenoalkylsulphinyl having 1 to 4 carbon atoms and 1 to 5 halogen atoms; sulphonyl; alkylsulphonyl having 1 to 4 carbon atoms; halogenoalkylsulphonyl having 1 to 4 carbon atoms and 1 to 5 halogen atoms; arylsulphonyl having 6 to 10 aryl carbon atoms, acyl, aryl, aryloxy, heteroaryl, heteroaryloxy, which may in turn carry one of the abovementioned substituents, and also the formimino radical -(CH)=N-O-alkyl;
and alkyl, alone or as part of a radical, denoting straight-chain or branched alkyl having 1 to 9 carbon atoms; alkenyl, alone or as part of a radical, denoting straight-chain or branched alkenyl having 2 to 20 carbon atoms; cycloalkyl denoting monocyclic, dicyclic and tricyclic cycloalkyl having 3 to 10 carbon atoms; aryl denoting phenyl or naphthyl; arylalkyl having 6 to 10 carbon atoms in the aryl moiety and 1 to 4 carbon atoms in the alkyl moiety, which may be straight-chain or branched; heteroaryl representing a 5- to 7-membered ring having 1 to 3 identical or different heteroatoms selected from oxygen, sulphur and nitrogen;
and optical isomers and racemates thereof.

4. Process for the preparation of the dioxomorpholines of the formula (I) according to Claim 3 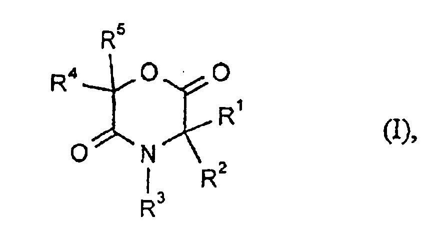 in which
the radicals R¹ to R⁵ have the meaning given under Claim 3,
**characterized in that**
a) open-chain depsipeptides of the formula (II) 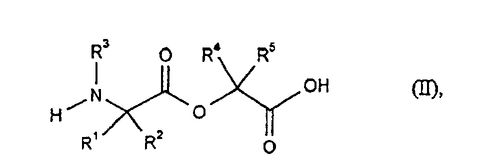 in which
the radicals R¹ to R⁵ have the abovementioned meaning,
are cyclized in the presence of a diluent and in the presence of a coupling reagent,
or
b) compounds of the formula (I) 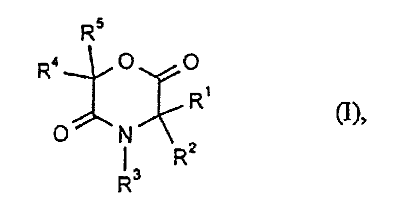 in which
R³ represents hydrogen and
R¹, R², R⁴ and R⁵ have the abovementioned meaning,
are reacted with compounds of the formula (III)
X-R³ (III),
in which
R³ represents straight-chain or branched alkyl, cycloalkyl, aralkyl or heteroarylalkyl, which can optionally be substituted,
X represents I, Cl or Br,
in the presence of a diluent and a base.

5. Open-chain depsipeptides of the formula (II) 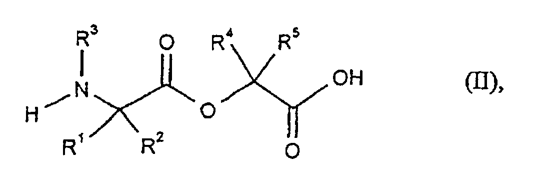 in which
R¹ to R⁵ have the meanings given in Claim 3.

6. Process for the preparation of the open-chain depsipeptides of the formula (II) 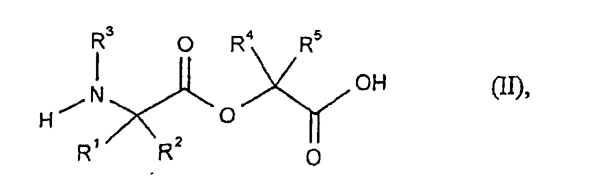 in which
R¹ to R⁵ have the meaning given in Claim 3
**characterized in that**
a) compounds of the formula (IV) 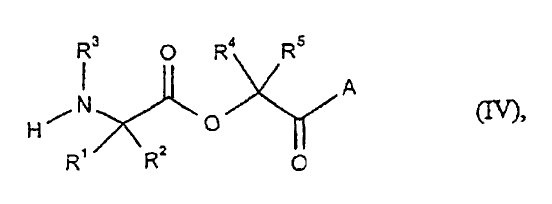 in which
A represents tert-butoxy,
R¹ to R⁵ have the abovementioned meanings,
are hydrolysed in the presence of a diluent and a proton acid,
or
b) compounds of the formula (V) 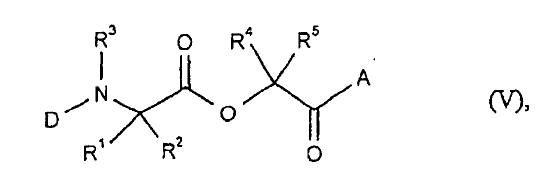 in which
A represents tert-butoxy,
D represents tert-butoxycarbonyl (-CO₂^{t}Bu),
R¹ to R⁵ have the abovementioned meanings,
are hydrolysed in the presence of a diluent and a proton acid.

7. Compounds of the formula (IV) 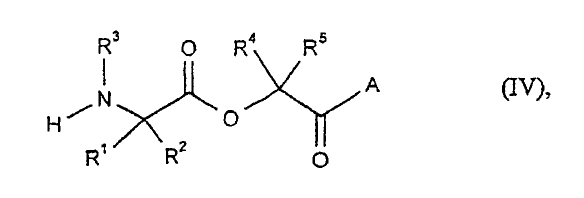 in which
A represents tert-butoxy,
R¹ to R⁵ have the meanings given in Claim 3,
with the proviso that if
R¹ and R⁴ represent hydrogen
R² is not hydrogen, straight-chain or branched alkyl, alkenyl, cycloalkyl, aryl, arylalkyl or heteroarylalkyl which are each optionally substituted.

8. Process for the preparation of compounds of the formula (IV) 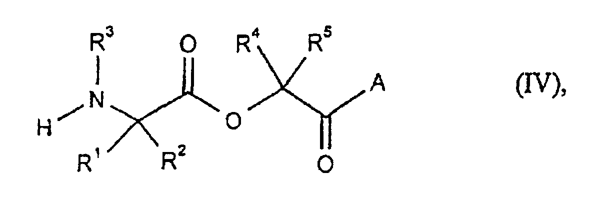 in which
A represents tert-butoxy,
R¹ to R⁵ have the meanings given in Claim 7,
**characterized in that** compounds of the formula (VI) 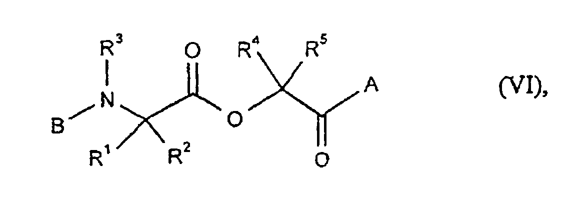 in which
A for tert-butoxy,
B for benzyl,
R¹ to R⁵ have the abovementioned meaning,
are hydrogenolysed in the presence of a diluent and a catalyst.

9. Compounds of the formula (V) 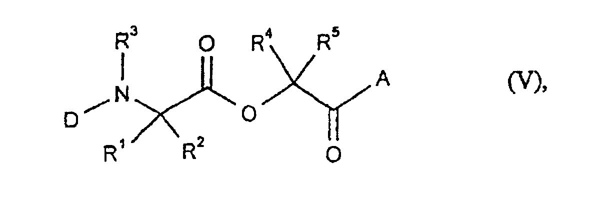 in which
A represents tert-butoxy,
D represents tert-butoxycarbonyl,
R¹ to R⁵ have the meanings given in Claim 3.

10. Process for the preparation of compounds of the formula (V) 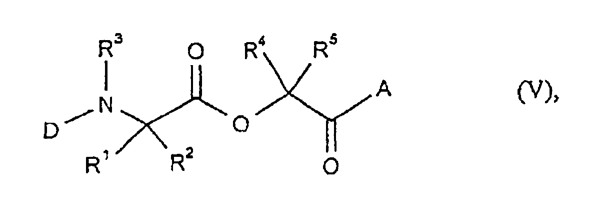 in which
A represents tert-butoxy,
D represents tert-butoxycarbonyl,
R¹ to R⁵ have the meanings given in Claim 3,
**characterized in that** compounds of the formula (VII) 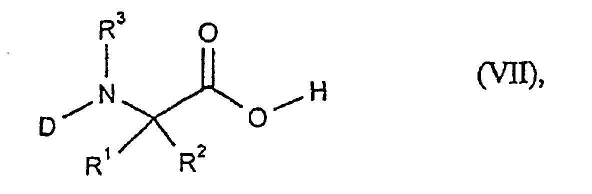 in which
D represents tert-butoxycarbonyl,
R¹ to R³ have the abovementioned meaning, in the form of their alkali metal salt, preferably their caesium salt, and an α-halogenocarboxylic acid of the formula (VIII) 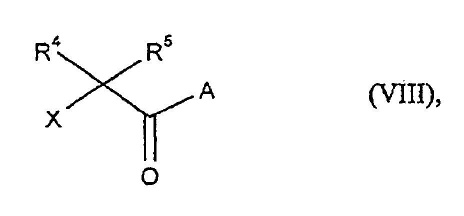
in which
A represents tert-butoxy,
X represents Cl or Br and
R⁴ and R⁵ have the abovementioned meaning,
are reacted in the presence of a diluent.

11. Compounds of the formula (VI) 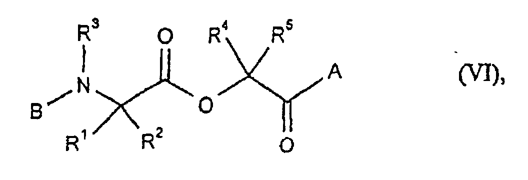 in which
A for tert-butoxy,
B represents benzyl,
R¹ to R⁵ have the meaning given in Claim 3,
with the proviso that if
R¹ and R⁴ represent hydrogen
R² is not hydrogen, straight-chain or branched alkyl, alkenyl, cycloalkyl, aryl, arylalkyl or heteroarylalkyl which are each optionally substituted.

12. Process for the preparation of compounds of the formula (VI) 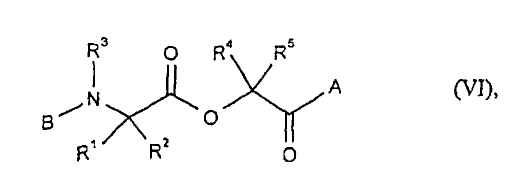 in which
A represents tert-butoxy,
B for benzyl and
R¹ to R⁵ have the meaning given in Claim 11,
**characterized in that** compounds of the formula (IX) 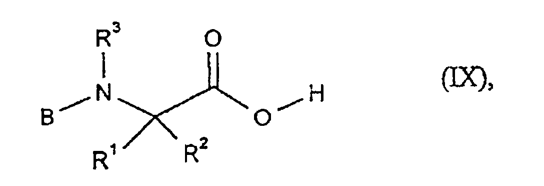 in which
B represents benzyl,
R¹ to R³ have the abovementioned meaning,
in the form of their alkali metal salt, preferably their caesium salt, are reacted with an α-halogenocarboxylic acid derivative of the formula (VIII) 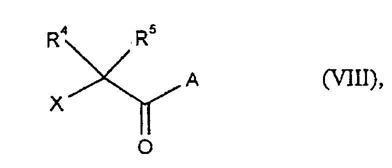 in which
X represents Cl or Br,
A for tert-butoxy,
R⁴ and R⁵ have the abovementioned meaning.

13. Endoparasiticidal compositions, **characterized in that** they contain at least one dioxomorpholine of the formula (I) according to Claim 3.

14. Process for the preparation of endoparasiticidal compositions, **characterized in that** dioxomorpholines of the formula (I) according to Claim 3 are mixed with extenders and/or surface-active agents.

## Revendications

1. Utilisation de dioxomorpholines de formule (I) 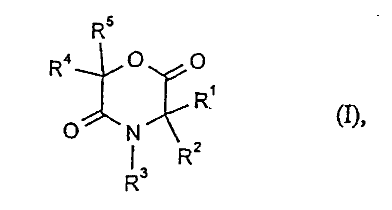 dans laquelle
R¹ et R² représentent, indépendamment l'un de l'autre, l'hydrogène, un groupe alkyle linéaire ou ramifié, un groupe alcényle, cycloalkyle, cycloalkylalkyle ainsi qu'un groupe aryle éventuellement substitué, des groupes arylalkyle, hétéroaryle, hétéroarylalkyle qui sont éventuellement substitués,
R¹ et R² forment ensemble un reste spirocyclique qui est éventuellement substitué,
R³ représente l'hydrogène, un groupe alkyle linéaire ou ramifié, des groupes cycloalkyle, aralkyle, aryle, hétéroaryle, hétéroarylalkyle qui sont éventuellement substitués,
R² et R³ forment conjointement avec les atomes auxquels ils sont liés un noyau pentagonal ou hexagonal qui peut être éventuellement substitué,
R⁴ et R⁵ représentent, indépendamment l'un de l'autre, l'hydrogène, un groupe alkyle linéaire ou ramifié, un groupe alcényle, cycloalkyle, cycloalkylalkyle, ainsi qu'un groupe aryle éventuellement substitué, des groupes arylalkyle, hétéroaryle, hétéroarylalkyle qui sont éventuellement substitués,
R⁴ et R⁵ forment ensemble un reste spirocyclique qui est éventuellement substitué,
ainsi que de leurs isomères optiques et de leurs racémates,
pour la préparation de compositions destinées à combattre des endoparasites en médecine et en médecine vétérinaire, les restes éventuellement substitués portant un ou plusieurs substituants identiques ou différents qui sont choisis dans le groupe : alkyle ayant 1 à 4 atomes de carbone, alkoxy ayant 1 à 4 atomes de carbone ; alkylthio ayant 1 à 4 atomes de carbone ; halogénalkyle ayant 1 à 4 atomes de carbone et 1 à 5 atomes d'halogènes identiques ou différents ; hydroxy ; halogène ; cyano ; nitro ; amino ; monoalkyl- et dialkylamino ayant 1 à 4 atomes de carbone par groupe alkyle ; alkylcarbonylamino ayant 1 à 4 atomes de carbone dans la partie alkyle ; benzoylamino, les restes alkylcarbonylamino et benzoylamino pouvant être substitués à chaque fois dans la partie acyle avec un halogène, un radical nitro, trifluorométhyle, trifluorométhoxy ou trifluorométhylthio ; carboxyle ; carbalkoxy ayant 2 à 4 atomes de carbone, alkylsulfinyle ayant 1 à 4 atomes de carbone, halogénalkylsulfinyle ayant 1 à 4 atomes de carbone et 1 à 5 atomes d'halogènes ; sulfonyle ; alkylsulfonyle ayant 1 à 4 atomes de carbone ; halogénalkylsulfonyle ayant 1 à 4 atomes de carbone et 1 à 5 atomes d'halogènes ; arylsulfonyle ayant 6 à 10 atomes de carbone, acyle, aryle, aryloxy, hétéroaryle, hétéroaryloxy pouvant porter chacun l'un des substituants mentionnés ci-dessus, ainsi que le reste formimino-(CH)=N-O-alkyle ;
et le terme alkyle seul ou comme partie d'un reste désigne un groupe alkyle linéaire ou ramifié ayant 1 à 9 atomes de carbone ; le terme alcényle seul ou comme partie d'un reste désigne un groupe alcényle linéaire ou ramifié ayant 2 à 20 atomes de carbone ; le terme cycloalkyle désigne un groupe cycloalkyle monocyclique, dicyclique ou tricyclique ayant 3 à 10 atomes de carbone ; le terme aryle désigne le groupe phényle ou naphtyle ; le terme arylalkyle présente 6 à 10 atomes de carbone dans la partie aryle et 1 à 4 atomes de carbone dans la partie alkyle qui peut être linéaire ou ramifiée ; le terme hétéroaryle désigne un noyau pentagonal à heptagonal ayant 1 à 3 hétéroatomes identiques ou différents choisis entre l'oxygène, le soufre et l'azote.

2. Utilisation de dioxomorpholines de formule (I) suivant la revendication 1,
formule dans laquelle
R¹ et R² désignent, indépendamment l'un de l'autre, l'hydrogène, un groupe alkyle linéaire ou ramifié ayant jusqu'à 8 atomes de carbone, un groupe halogénalkyle, hydroxyalkyle, alcanoyloxyalkyle, alkoxyalkyle, aryloxyalkyle, mercaptoalkyle, alkylthioalkyle, alkylsulfinylalkyle, alkylsulfonylalkyle, carboxyalkyle, alkoxycarbonylalkyle, arylalkoxycarbonylalkyle, carbamoylalkyle, aminoalkyle, alkylaminoalkyle, dialkylaminoalkyle, guanidinoalkyle, qui peut être substitué, le cas échéant, par un ou deux restes benzyloxycarbonyle ou par un, deux, trois ou quatre restes alkyle, un groupe alkoxycarbonylaminoalkyle, 9-fluorénylméthoxycarbonyl(Fmoc)-aminoalkyle, alcényle, cycloalkyle, cycloalkylalkyle, de même qu'un groupe aryle, arylalkyle, hétéroaryle, hétéroarylalkyle éventuellement substitué,
R¹ et R² forment ensemble un reste spirocyclique,
R³ représente l'hydrogène, un groupe alkyle linéaire ou ramifié, cycloalkyle, aralkyle, aryle, hétéroaryle, hétéroarylalkyle, qui sont éventuellement substitués,
R² et R³ forment conjointement avec les atomes auxquels ils sont liés un noyau pentagonal ou hexagonal qui peut être éventuellement substitué,
R⁴ et R⁵ représentent, indépendamment l'un de l'autre, l'hydrogène, un groupe alkyle linéaire ou ramifié ayant jusqu'à 8 atomes de carbone, un groupe halogénalkyle, hydroxyalkyle, alcanoyloxyalkyle, alkoxyalkyle, aryloxyalkyle, mercaptoalkyle, alkylthioalkyle, alkylsulfinylalkyle, alkylsulfonylalkyle, carboxyalkyle, alkoxycarbonylalkyle, arylalkoxycarbonylalkyle, carbamoylalkyle, aminoalkyle, alkylaminoalkyle, dialkylaminoalkyle, guanidinoalkyle, pouvant porter, le cas échéant, comme substituants un ou deux restes benzyloxycarbonyle ou un, deux, trois ou quatre restes alkyle, un groupe alkoxycarbonylaminoalkyle, 9-fluorénylméthoxycarbonyl(Fmoc)aminoalkyle, alcényle, cycloalkyle, cycloalkylalkyle, de même qu'un groupe aryle, arylalkyle, hétéroaryle, hétéroarylalkyle éventuellement substitué,
R⁴ et R⁵ forment ensemble un reste spirocyclique,
ainsi que de leurs isomères optiques ou de leurs racémates.

3. Dioxomorpholines de formule (I) 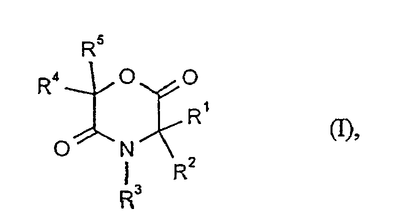 dans laquelle
R¹ représente l'hydrogène, un groupe alkyle linéaire ou ramifié ayant jusqu'à 8 atomes de carbone, halogénalkyle, hydroxyalkyle, alcanoyloxyalkyle, alkoxyalkyle, aryloxyalkyle, mercapto-alkyle, alkylthioalkyle, alkylsulfinylalkyle, alkylsulfonylalkyle, carboxyalkyle, alkoxycarbonylalkyle, arylalkoxycarbonylalkyle, carbamoylalkyle, aminoalkyle, alkylaminoalkyle, dialkylaminoalkyle, guanidinoalkyle pouvant porter, le cas échéant, comme substituants un ou deux restes benzyloxycarbonyle ou un, deux, trois ou quatre restes alkyle, un groupe alkoxycarbonylaminoalkyle, 9-fluorénylméthoxycarbonyl(Fmoc)aminoalkyle, alcényle, cycloalkyle, cyclo-alkylalkyle, ainsi qu'un groupe aryle, arylalkyle, hétéroaryle, hétéroarylalkyle éventuellement substitué,
R² représente l'hydrogène, un groupe alkyle linéaire ou ramifié ayant jusqu'à 8 atomes de carbone, un groupe halogénalkyle, hydroxyalkyle, alcanoyloxyalkyle, alkoxyalkyle, aryloxyalkyle, mercaptoalkyle, alkylthioalkyle, alkylsulfinylalkyle, alkylsulfonylalkyle, carboxyalkyle, alkoxycarbonylalkyle, arylalkoxycarbonylalkyle, carbamoylalkyle, aminoalkyle, alkylaminoalkyle, dialkylaminoalkyle, guanidinoalkyle pouvant porter, le cas échéant, comme substituants un ou deux restes benzyloxycarbonyle ou un, deux, trois ou quatre restes alkyle, un groupe alkoxycarbonylaminoalkyle, 9-fluorénylméthoxycarbonyl(Fmoc)aminoalkyle, alcényle, cycloalkyle, cyclo-alkylalkyle, ainsi qu'un groupe aryle, arylalkyle, hétéroaryle, hétéroarylalkyle éventuellement substitué,
R¹ et R² peuvent former ensemble un reste spirocyclique,
R³ représente un groupe alkyle linéaire ou ramifié, des groupes cycloalkyle, aralkyle, aryle, hétéroaryle, hétéroarylalkyle, qui sont éventuellement substitués,
R⁴ représente l'hydrogène, un groupe alkyle linéaire ou ramifié ayant jusqu'à 8 atomes de carbone, un groupe halogénalkyle, hydroxyalkyle, alcanoyloxyalkyle, alkoxyaryle, aryloxyalkyle, mercaptoalkyle, alkylthioalkyle, alkylsulfinylalkyle, alkylsulfonylalkyle, alkoxycarbonylalkyle, arylalkoxycarbonylalkyle, carbamoylalkyle, aminoalkyle, alkylaminoalkyle, dialkylaminoalkyle, guanidinoalkyle pouvant porter, le cas échéant, comme substituants un ou deux restes benzyloxycarbonyle ou bien un, deux, trois ou quatre restes alkyle, un groupe alkoxycarbonylaminoalkyle, 9-fluorénylméthoxycarbonyl (Fmoc)aminoalkyle, alcényle, cycloalkyle, cycloalkylalkyle, ainsi qu'un groupe aryle, arylalkyle, hétéroaryle, hétéroarylalkyle éventuellement substitué,
R⁵ représente l'hydrogène, un groupe alkyle linéaire ou ramifié ayant jusqu'à 8 atomes de carbone, un groupe halogénalkyle, hydroxyalkyle, alcanoyloxyalkyle, alkoxyalkyle, aryloxyalkyle, mercaptoalkyle, alkylthioalkyle, alkylsulfinylalkyle, alkylsulfonylalkyle, alkoxycarbonylalkyle, arylalkoxycarbonylalkyle, carbamoylalkyle, aminoalkyle, alkylaminoalkyle, dialkylaminoalkyle, guanidinoalkyle pouvant porter, le cas échéant, comme substituants un ou deux restes benzyloxycarbonyle ou un, deux, trois ou quatre restes alkyle, un groupe alkoxycarbonylaminoalkyle, 9-fluorénylméthoxycarbonyl (Fmoc)aminoalkyle, alcényle, cycloalkyle, cycloalkylalkyle, ainsi qu'un groupe aryle, arylalkyle, hétéroaryle, hétéroarylalkyle éventuellement substitué,
R⁴ et R⁵ forment ensemble un reste spirocyclique,
à l'exception des composés dans lesquels, simultanément
a)
R¹ désigne l'hydrogène,
R² désigne l'hydrogène, un groupe méthyle, benzyle ou isopropyle et
R³ désigne un groupe méthyle ou un groupe phényle à substituant alkyle,
ou
b)
R¹ est l'hydrogène,
R² est un groupe sec.-butyle ou isobutyle,
R³ est un groupe méthyle,
R⁴ est l'hydrogène et
R⁵ est un groupe isopropyle,
ou bien
c)
R¹ est l'hydrogène,
R² est un groupe isopropyle,
R³ est l'hydrogène,
R⁴ est l'hydrogène,
R⁵ est un groupe isopropyle,
les restes éventuellement substitués portant un ou plusieurs substituants identiques ou différents qui sont choisis dans le groupe : alkyle ayant 1 à 4 atomes de carbone, alkoxy ayant 1 à 4 atomes de carbone ; alkylthio ayant 1 à 4 atomes de carbone ; halogénalkyle ayant 1 à 4 atomes de carbone et 1 à 5 atomes d'halogènes identiques ou différents ; hydroxy ; halogène ; cyano ; nitro ; amino ; monoalkyl- et dialkylamino ayant 1 à 4 atomes de carbone par groupe alkyle ; alkylcarbonylamino ayant 1 à 4 atomes de carbone dans la partie alkyle ; benzoylamino, les restes alkylcarbonylamino et benzoylamino pouvant, dans chaque cas, être substitués dans la partie acyle avec un halogène, un radical nitro, trifluorométhyle, trifluorométhoxy ou trifluorométhylthio ; carboxyle ; carbalkoxy ayant 2 à 4 atomes de carbone, alkylsulfinyle ayant 1 à 4 atomes de carbone, halogénalkylsulfinyle ayant 1 à 4 atomes de carbone et 1 à 5 atomes d'halogènes ; sulfonyle ; alkylsulfonyle ayant 1 à 4 atomes de carbone ; halogénalkylsulfonyle ayant 1 à 4 atomes de carbone et 1 à 5 atomes d'halogènes ; arylsulfonyle ayant 6 à 10 atomes de carbone dans la partie aryle, acyle, aryle, aryloxy, hétéroaryle, hétéroaryloxy pouvant porter chacun l'un des substituants mentionnés ci-dessus, ainsi que le reste formimino-(CH)=N-O-alkyle ;
et le terme alkyle seul ou comme partie d'un reste désigne un groupe alkyle linéaire ou ramifié ayant 1 à 9 atomes de carbone ; le terme alcényle seul ou comme partie d'un reste désigne un groupe alcényle linéaire ou ramifié ayant 2 à 20 atomes de carbone ; le terme cycloalkyle désigne un groupe cycloalkyle monocyclique, dicyclique et tricyclique ayant 3 à 10 atomes de carbone ; le terme aryle désigne un groupe phényle ou naphtyle ; le terme arylalkyle désigne un groupe ayant 6 à 10 atomes de carbone dans la partie aryle et 1 à 4 atomes de carbone dans la partie alkyle qui peut être linéaire ou ramifiée ; le terme hétéroaryle désigne un noyau pentagonal à heptagonal ayant 1 à 3 hétéroatomes identiques ou différents choisis entre oxygène, soufre et azote ;
ainsi que leurs isomères optiques et leurs racémates.

4. Procédé de production des dioxomorpholines de formule (I) suivant la revendication 3 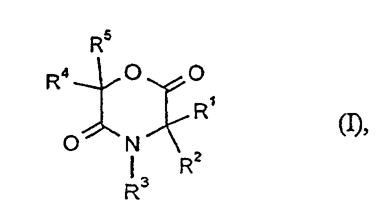 dans laquelle
les restes R¹ à R⁵ ont la définition indiquée dans la revendication 3,
**caractérisé en ce que**
a) on cyclise des depsipeptides à chaîne ouverte de formule (II) 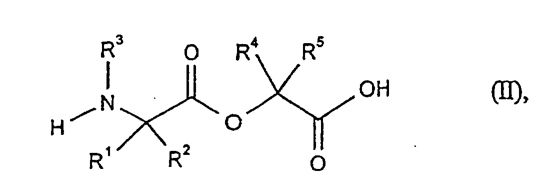 dans laquelle
les restes R¹ à R⁵ ont la définition indiquée ci-dessus,
en présence d'un diluant et en présence d'un réactif de couplage,
ou bien
b) on fait réagir des composés de formule (I) 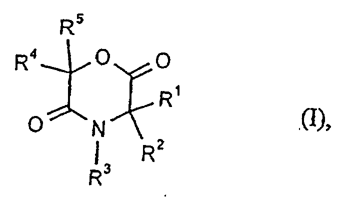 dans laquelle
R³ représente l'hydrogène et
R¹, R², R⁴ et R⁵ ont la définition indiquée ci-dessus avec des composés de formule (III)
X-R³ (III)
dans laquelle
R³ est un groupe alkyle linéaire ou ramifié, un groupe cycloalkyle, un groupe aralkyle ou un groupe hétéroarylalkyle qui peuvent éventuellement être substitués,
X représente I, Cl ou Br,
en présence d'un diluant et d'une base.

5. Depsipeptides à chaîne ouverte de formule (für) 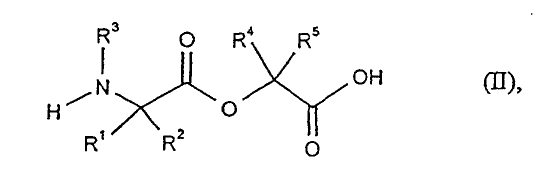 dans laquelle
R¹ à R⁵ ont les définitions indiquées dans la revendication 3.

6. Procédé de production des depsipeptides à chaîne ouverte de formule (II) 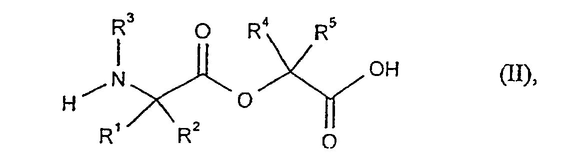 dans laquelle
R¹ à R⁵ ont la définition indiquée dans la revendication 3,
**caractérisé en ce que**
a) on saponifie des composés de formule (IV) 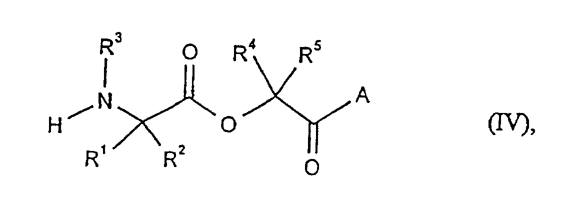 dans laquelle
A est un groupe tertio-butoxy,
R¹ à R⁵ ont les définitions indiquées ci-dessus,
en présence d'un diluant et d'un acide protonique,
ou bien
b) on saponifie des composés de formule (V) 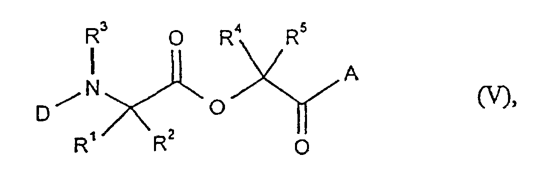 dans laquelle
A est un groupe tertio-butoxy,
D est un groupe tertio-butoxycarbonyl(-CO₂^{t}Bu),
R¹ à R⁵ ont les définitions indiquées ci-dessus,
en présence d'un diluant et d'un acide protonique.

7. Composés de formule (IV) 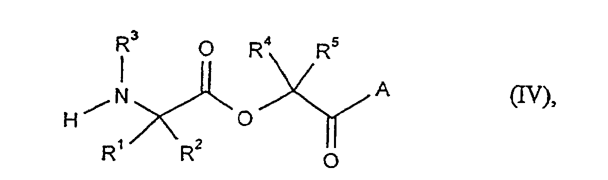 dans laquelle
A est un groupe tertio-butoxy,
R¹ à R⁵ ont les définitions indiquées dans la revendication 3,
sous réserve que, lorsque
R¹ et R⁹ représentent l'hydrogène,
R² ne représente pas l'hydrogène, un groupe alkyle linéaire ou ramifié, alcényle, cycloalkyle, aryle, arylalkyle ou hétéroarylalkyle dont chacun est éventuellement substitué.

8. Procédé de production de composés de formule (IV) 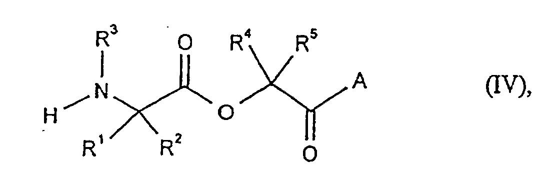 dans laquelle
A est un groupe tertio-butoxy,
R¹ à R⁵ ont les définitions indiquées dans la revendication 7,
**caractérisé en ce qu'**on hydrogénolyse des composés de formule (VI) 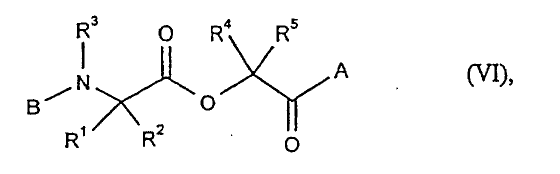 dans laquelle
A est un groupe tertio-butoxy,
B est un groupe benzyle,
R¹ à R⁵ ont la définition indiquée ci-dessus,
en présence d'un diluant et d'un catalyseur.

9. Composés de formule (V) 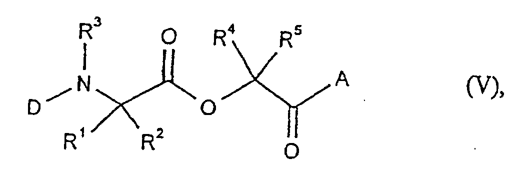 dans laquelle
A est un groupe tertio-butoxy,
D est un groupe tertio-butoxycarbonyle,
R¹ à R⁵ ont les définitions indiquées dans la revendication 3.

10. Procédé de production de composés de formule (V) 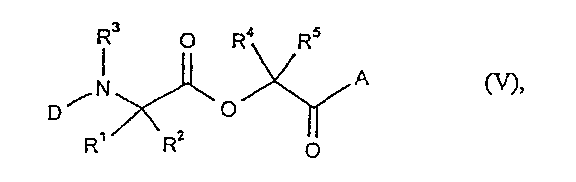 dans laquelle
A est un groupe tertio-butoxy,
D est un groupe tertio-butoxycarbonyle,
R¹ à R⁵ ont les définitions indiquées dans la revendication 3,
**caractérisé en ce qu'**on fait réagir des composés de formule (VII) 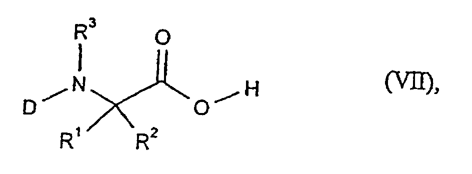 dans laquelle
D est un groupe tertio-butyloxycarbonyle,
R¹ à R³ ont la définition indiquée ci-dessus,
sous forme de leur sel de métal alcalin, de préférence de leur sel de césium, et un acide α-halogénocarboxylique de formule (VIII) 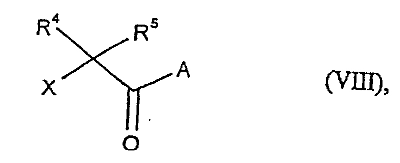 dans laquelle
A est un groupe tertio-butoxy,
X représente Cl ou Br et
R⁴ et R⁵ ont la définition indiquée ci-dessus,
en présence d'un diluant.

11. Composés de formule (VI) 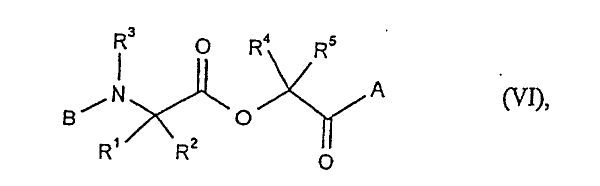 dans laquelle
A est un groupe tertio-butoxy,
B est un groupe benzyle,
R¹ à R⁵ ont la définition indiquée dans la revendication 3,
sous réserve que
lorsque R¹ et R⁴ représentent l'hydrogène,
R² ne représente pas l'hydrogène, un groupe alkyle linéaire ou ramifié, alcényle, cycloalkyle, aryle, arylalkyle ou hétéroarylalkyle dont chacun est éventuellement substitué.

12. Procédé de production de composés de formule (VI) 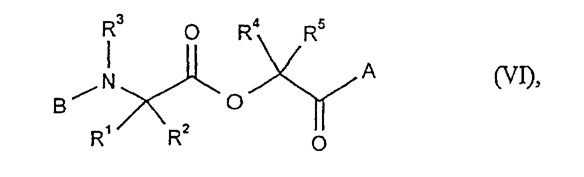 dans laquelle
A est un groupe tertio-butoxy,
B est un groupe benzyle ainsi que
R¹ à R⁵ ont la définition indiquée dans la revendication 11,
**caractérisé en ce qu'**on fait réagir des composés de formule (IX) 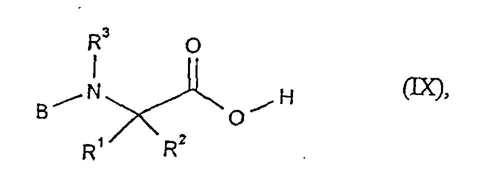 dans laquelle
B est un groupe benzyle,
R¹ à R³ ont la définition indiquée ci-dessus,
sous forme de leur sel de métal alcalin, de préférence leur sel de césium, avec un dérivé d'acide α-halogénocarboxylique de formule (VIII) 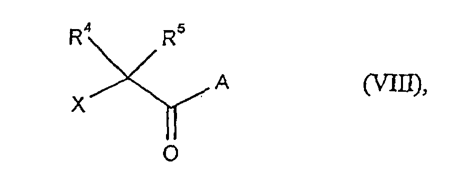 dans laquelle
X représente Cl, Br,
A est un groupe tertio-butoxy,
R⁴ et R⁵ ont la définition indiquée ci-dessus.

13. Compositions endoparasiticides, **caractérisées par** une teneur en au moins une dioxomorpholine de formule (I) suivant la revendication 3.

14. Procédé de préparation de compositions endoparasiticides, **caractérisé en ce qu'**on mélange des dioxomorpholines de formule (I) suivant la revendication 3, avec des diluants et/ou des agents tensio-actifs.
